# EUROPEAN PATENT SPECIFICATION

(11) **EP 4 085 067 B1**
(45) Date of publication and mention of the grant of the patent: **02.09.2026**
(21) Application number: 20839350.4
(22) Date of filing: 29.12.2020
(51) Int. Cl.: C07K 14/47, G01N 33/53

(54) **METHOD FOR DETERMINING 5-METHYLCYTOSINE CONFIGURATIONS IN DNA**
VERFAHREN ZUR BESTIMMUNG VON 5-METHYLCYTOSIN-KONFIGURATIONEN IN DNA
PROCÉDÉ POUR DÉTERMINER DES CONFIGURATIONS 5-MÉTHYLCYTOSINE DANS L'ADN

(30) Priority: 30.12.2019 EP 19220082
(43) Date of publication of application: 09.11.2022
(73) Proprietor: Technische Universität Dortmund, 44227 Dortmund (DE)
(72) Inventor: SUMMERER, Daniel, 44137 Dortmund (DE); BUCHMULLER, Benjamin, 44379 Dortmund (DE)
(74) Representative: Maiwald GmbH
(86) International application number: PCT/EP2020/087979
(87) International publication number: WO 2021/136780

(56) References cited:
- WO-A2-2016/210124
- T. C. GALVAO ET AL: "Structure-specific binding of MeCP2 to four-way junction DNA through its methyl CpG-binding domain", NUCLEIC ACIDS RESEARCH, vol. 33, no. 20, 27 November 2005 (2005-11-27), pages 6603 - 6609, XP055704860, ISSN: 0305-1048, DOI: 10.1093/nar/gki971
- NAOYUKI FUJITA ET AL: "Mechanism of Transcriptional Regulation by Methyl-CpG Binding Protein MBD1", MOLECULAR AND CELLULAR BIOLOGY, vol. 20, no. 14, 1 January 2000 (2000-01-01), pages 5107 - 5118, XP055704784
- S KUDO: "Heterogeneity in residual function of MeCP2 carrying missense mutations in the methyl CpG binding domain", JOURNAL OF MEDICAL GENETICS, vol. 40, no. 7, 1 July 2003 (2003-07-01), pages 487 - 493, XP055705067, DOI: 10.1136/jmg.40.7.487

## Description

### FIELD OF THE INVENTION

The present invention relates to an isolated Methyl-CpG binding domain (MBD) variant comprising an MBD core domain that has at least 85 % sequence homology relative to any one of SEQ ID Nos. 1-28 and comprising at least one amino acid substitution relative to the corresponding wildtype MBD in at least one of the positions corresponding to positions 12, 25, 26, and 37 in SEQ ID NO:1. Furthermore, the invention relates to a conjugate comprising the isolated MBD variant and to the use of the isolated MBD variant or the conjugate for the determination of the methylation state of cytosine residues and/or oxidation state of 5-methylated cytosine residues in a CpG dinucleotide of interest and its complement in a DNA molecule or for the enrichment of DNA molecules comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC). Finally, the invention relates to specific methods for the determination of the methylation state of cytosine residues and/or oxidation state of the methyl group of 5-methylated cytosine residues as described above or for the enrichment of DNA molecules as described above.

### BACKGROUND OF THE INVENTION

Modifications of the canonical DNA nucleobases with small chemical groups such as a methyl or carboxy group, alter the physicochemical properties of the DNA major groove and in that way, the lead interface at which DNA-binding proteins such as transcription factors access the genetic information. This 'epigenetic' mechanism is used in many organisms to naturally control gene expression. As an example, 5-methylcytosine (mC), a pervasive modification of cytosine in mammalian genomes (4-5 mol% of all deoxycytidines), is decisively absent from activating regulatory elements such as gene promoters and enhancers.

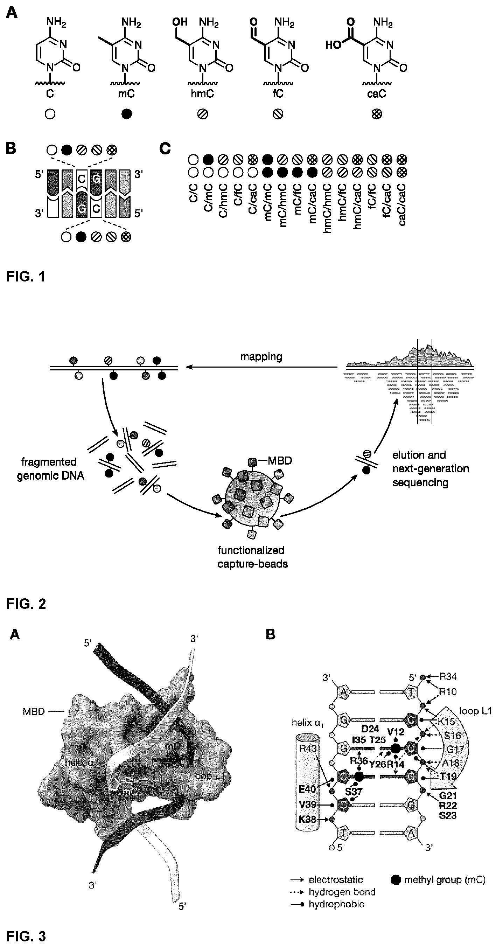

In human and mouse, mC is almost exclusively limited to the deoxycytidine-phosphate-deoxyguanosine (CpG) dinucleotide context, with both deoxycytidines in the DNA double-strand being modified concurrently. The mC landscape is actively shaped throughout development and differentiation by DNA methyltransferases such as DNMT3a and DNMT3b, which establish mC at non-methylated CpGs, or DNMT1, which re-consolidates hemi-methylated CpGs after semi-conservative DNA replication. Furthermore, ten-eleven translocation (TET) dioxygenases iteratively oxidize mC to 5-hydroxymethyl-(hmC), 5-formyl- (fC), and 5-carboxycytosine (caC) (FIG. 1A), eventually promoting demethylation by base-excision repair.

The longevity of some of these oxidized mC species (collectively referred to as oxi-mC) at non-random sites in the mammalian genome, their effect on protein binding, DNA flexibility and their chemical reactivity, has established oxi-mC as additional epigenetic marks in genomes with biological functions that differ from the ones of mC.

In vitro, TET enzymes oxidize modified and hemi-modified CpGs in a non-processive manner, independent of the oxi-mC species on the complementary DNA strand. Thus, different mC and oxi-mC species likely co-exist at any CpG dinucleotide (FIG. 1B) in one of 15 conceivable, symmetric and asymmetric configurations (FIG. 1C). Yet, it is unknown which of these configurations exist *in vivo* and where they occur in the genome.

Direct sequencing of genomic DNA indicates that of all CpGs that contain hmC (6.2 mol%) only 21 % are symmetrically modified [1, 2]. Likewise, the mean difference of hmC or fC levels at CpGs in CpG-rich regions is 43-46 % (for mC this is 18 %) [3], and the genome-wide average correlation of oxi-mC levels within CpGs as low as 1-15 % (82 % for mC) [4].

Current technologies cannot resolve individual oxi-mC configurations because the read-out of the deoxycytidine modifications is based on bisulfite conversion and therefore binary. It is thus only possible to distinguish two oxi-mC species at once. Given a DNA molecule cannot be converted more than once, the fractional composition of each modified nucleobase must be inferred from the combination of different (bulk) sequencing experiments by probabilistic models. Consequently, the original oxi-mC configuration in single double-stranded DNA molecules cannot be determined.

Also, sequential affinity-enrichment with commercially available protein receptors against single oxi-mC [5, 6] is unsuitable to identify the oxi-mC composition at CpGs as neighboring modifications on the same DNA fragment cannot be discriminated. Antibodies, for example, have the additional drawback that they do not provide strand-selectivity.

WO 2016210124 A2 describes human methyl binding domain 2 (hMBD2) variants which bind a DNA sequence with a single methylated CpG site with high affinity.

Thus, the lack of suitable binders to capture defined oxi-mC configuration at CpGs impedes further understanding of their biological function in the mammalian genome.

### SUMMARY OF THE INVENTION

The invention is defined by the appended claims.

Surprisingly, the inventors found specific engineered Methyl-CpG binding domains (MBD) that preferably provide differential interactions with different mC and/or oxi-mC configurations at (single) CpGs in a DNA molecule. Based on these findings, the inventors created molecular probes on the basis of these engineered Methyl-CpG binding domains which preferably resolve the precise configuration of oxi-mC species across the DNA double-strand, more preferably at the single-molecule level. These molecular probes or engineered Methyl-CpG binding domains can find application in diverse analytical formats, ranging from affinity enrichment over imaging, flow cytometry, improved direct high-throughput sequencing methods and others. For example, they can enable affinity enrichment assays with MBD-functionalized capture beads that enable retrieving double-stranded DNA molecules with defined oxi-mC configurations from complex genomic DNA samples (FIG. 2). In another example, they can be used for mapping CpGs with distinct configurations, and ultimately allow correlating such genome-wide maps with known (epi-)genetic features to reveal biological functions.

Therefore, in a first aspect, the invention relates to an isolated Methyl-CpG binding domain (MBD) variant, comprising an MBD core domain that has at least 85 % sequence homology relative to any one of SEQ ID Nos. 1-28, and comprising at least one amino acid substitution relative to the corresponding wildtype MBD in at least one of the positions corresponding to positions 12, 25, 26 and 37 in SEQ ID NO:1.

The inventors have found that these positions are involved with base recognition and binding and/or are located in the three-dimensional structure such as to contact the cytosine base of the bound DNA molecule. While in the following reference is made to the positions relative to SEQ ID NO:1, it is understood that positional numbering can also be based on any one of SEQ ID Nos. 2-28, in particular in any one of SEQ ID Nos. 1-5. Such embodiments are thus also encompassed by the present invention.

In various embodiments, the variant comprises two or more of the above-mentioned amino acid substitutions, such as 1, 2, 3, or 4. In various embodiments, the variant comprises at least 2, 3 or 4 amino acid substitutions. These are preferably selected from substitutions in positions 12, 25, 26, and 37 or, in case SEQ ID NO:3 is used as a reference, 12, 25, 27, and 37. In embodiments, where the variant comprises more than 4 substitutions, the 5^{th} and subsequent substitutions may occur in positions 19, 21, 22, 23, 38 and 39. In embodiments, where the variant comprises more than 12 substitutions, the additional substitutions may be selected from those at positions 14, 24, 36, and 40. In various embodiments, the additional substitutions may be selected from amino acid substitutions relative to the corresponding wildtype MBD in at least one of the positions corresponding to positions 14, 19, 21, 22, 23, 24, 27, 29, 31, 33, 35, 36, 38, 39, 40, and 45 in SEQ ID NO:1. In various embodiments, the variant comprises two or more of the above-mentioned amino acid substitutions, such as 3, 4, 5, 6, 7, 8, 9, 10, 11, 12, 13, 14, 15, 16, 17, 18, 19 or all 20. In some embodiments, the variant does not comprise any other modifications, in particular no other amino acid substitutions, outside of the positions listed herein.

The at least one, preferably at least two or at least three, amino acid substitution(s) in at least one, preferably in at least two or at least three, of the positions corresponding to positions 12, 25, 26 and 37 is selected from 12V, 12T, 12A, 12R, 12D, 12L, 12P, 25T, 25A, 25C, 25L, 25Y, 25P, 25S, 26T, 26S, 26F, 26L, 26D, 26V, 26Q, 26M, 37N, 37K, 37Q, 37R, 37V and 37F, preferably 12V, 12T, 12A, 12R, 25T, 25A, 25C, 25L, 26T, 26S, 26F, 26L, 26Q, 37N, 37K, 37Q, 37R and 37V, most preferably 12V, 12T, 25T, 25A, 25C, 26T, 26S, 26L, 26Q, 37N, 37R, and 37K using the positional numbering of SEQ ID NO:1.

In various embodiments, said at least one amino acid substitution is selected from 12T, 12A, 12V, 12R, 25T, 25A, 25C, 25L, 26T, 26S, 26F, 26L, 27F, 36C, 37N, 37K, 37Q, 37R, 37V and 37C, preferably 12T, 12A, 12V, 12R, 25I, 25T, 25A, 25C, 25L, 26T, 26S, 26F, 26L, 37N, 37K, 37Q, 37R and 37V, more preferably 12T, 25T, 25A, 26T, 37N and 37K using the positional numbering of SEQ ID NO:1.

In various embodiments, said at least one amino acid substitution in at least one of the positions corresponding to positions 12, 25, 26, and 37 is selected from 12V, 12T, 12A, 12R, 12D, 12L, 12P, 25T, 25A, 25C, 25L, 25Y, 25P, 25S, 26T, 26S, 26F, 26L, 26D, 26V, 26Q, 26M, 37N, 37K, 37Q, 37R, 37V and 37F, preferably 12V, 12T, 12A, 12R, 12D, 12L, 12P, 25T, 25A, 25C, 25L, 25Y, 25P, 25S, 26T, 26S, 26F, 26L, 26D, 26V, 26Q, 26M, 37N, 37K, 37Q, 37R, 37V and 37F, more preferably 12V, 12T, 12A, 12R, 25T, 25A, 25C, 25L, 26T, 26S, 26F, 26L, 26Q, 37N, 37K, 37Q, 37R and 37V, most preferably 12V, 12T, 25T, 25A, 25C, 26T, 26S, 26L, 26Q, 37N, 37R, and 37K using the positional numbering of SEQ ID NO:1.

In various embodiments, said at least one amino acid substitution is selected from the group consisting of 12V, 12T, 12A, 12R, 12D, 12L, 12P, 25T, 25A, 25C, 25L, 25Y, 25P, 25S, 26T, 26S, 26F, 26L, 26D, 26V, 26Q, 26M, 37N, 37K, 37Q, 37R, 37V and 37F, preferably 12T, 12A, 12R, 12D, 12L, 12P, 25T, 25A, 25C, 25L, 25Y, 25P, 25S, 26T, 26S, 26F, 26L, 26D, 26V, 26Q, 26M, 37N, 37K, 37Q, 37R, 37V, and 37F, more preferably 12T, 12A, 12R, 12D, 12L, 12P, 25T, 25A, 25C, 25L, 25Y, 25P, 26T, 26S, 26F, 26L, 26D, 26V, 26Q, 26M, 37N, 37K, 37Q, 37R, 37V, and 37F using the positional numbering of SEQ ID NO:1.

In various embodiments, the isolated MBD variant comprises at least two, preferably at least three, amino acid substitutions selected from 12V, 12T, 12A, 12R, 12D, 12L, 12P, 25T, 25A, 25C, 25L, 25Y, 25P, 25S, 26T, 26S, 26F, 26L, 26D, 26V, 26Q, 26M, 37N, 37K, 37Q, 37R, 37V and37F, using the positional numbering of SEQ ID NO:1.

In various embodiments, the isolated MBD variant comprises at least one, preferably at least two, amino acid substitution selected from 12V, 12T, 12A, 12R, 12D, 12L, 12P, 25T, 25A, 25C, 25L, 25Y, 25P, 25S, 26T, 26S, 26F, 26L, 26D, 26V, 26Q, 26M, 37N, 37K, 37Q, 37R, 37V, and 37F using the positional numbering of SEQ ID NO:1; and
(a) at least one amino acid substitution in at least one of the positions corresponding to positions 19, 21, 35, 38, and 40 in SEQ ID NO:1.

In various embodiments, wherein at least one amino acid substitution is in at least one of the positions corresponding to positions 27, 35, and 36, it is selected from 27F, 35L, 36C.

In various embodiments, wherein at least one amino acid substitution is in at least one of the positions corresponding to positions 27, 29, 31, 33, 35, 36 and 45, it is selected from 27F, 29L, 31A, 31D, 31H, 33E, 35L, 36C, and 45L, such as 27F, 29L, 31A, 31D, 31H, 33E, and 45L, such as 27F, 29L, 31A, 31D, 31H, 33E, 35L, 36C, and 45L.

In various embodiments, the isolated MBD variant comprises any two, three, four or five, of the substitutions set forth in the following sets of substitutions:
(1) 12T, 25T, 26T, 37K;
(2) 12T, 25A, 37N;
(3) 25C, 26S, 37Q;
(4) 12A, 25C, 26F, 37R;
(5) 25L, 26T, 37R;
(6) 12V, 26L, 37R;
(7) 12R, 37V;
(8) 12T, 25T, 26Q, 37K;
(9) 12T, 25C, 37N;
(10) 12T, 25A, 26M, 37N;
(11) 12D, 25C, 37N;
(12) 12A, 25L, 26M, 37R;
(13) 12L, 25Y, 27F, 37F;
(14) 12L, 25A, 26D, 31D:
(15) 12P, 25P, 26V, 31A;
(16) 12L, 25S, 26Q, 33E;
(17) 12A, 19C, 25C, 26M, 37N;
(18) 12T, 25A, 31H, 37N;
(19) 12T, 25A, 37N, 45L; or
(20) 12T, 25C, 29L, 35L, 37N.

In various embodiments, the sets of substitutions (1)-(6), (8)-(12) and (17)-(20) are relative to SEQ ID NO:5 (hMeCP2); set of substitution (13) is relative to SEQ ID NO:3 (hMBD3); and sets of substitutions (7) and (14)-(16) are relative to SEQ ID NO:2 (hMBD2). This means that the remainder of the MBD core sequence, also it may contain further substitutions as disclosed herein, is derived from the given template sequence. In various embodiments, it may contain only 1, 2 or 3 additional substitutions, in some embodiments no additional substitutions besides those recited in the set of substitutions are present.

In various embodiments, the MBD core domain comprises any one or more, preferably at least 2, more preferably at least 3, most preferably all 4, of the amino acids 14R/K, 24D/E, 36R/K and 40E/Q/D using the positional numbering of SEQ ID NO:1. These amino acids typically correspond to those at the same position in the wildtype MBD and are in some embodiments invariable.

In various other embodiments, the MBD core domain comprises any one or more, preferably at least 2, more preferably at least 3, most preferably all 4, of the amino acids 14R/K, 24D/E, 36R/K and 40E/Q/D/S using the positional numbering of SEQ ID NO:1. These amino acids typically correspond to those at the same position in the wildtype MBD and are in some embodiments invariable.

In various other embodiments, the MBD core domain comprises any one or more, preferably at least 2, more preferably at least 3, most preferably all 4, of the amino acids 14R/K/T, 24D/E/H, 36R/E/S/K and 40E/Q/S, preferably any one or more of the amino acids 14R, 24D, 36R and 40E/Q/S, using the positional numbering of SEQ ID NO:1. These amino acids typically correspond to those at the same position in the wildtype MBD and are in some embodiments invariable.

In various embodiments, the MBD core domain comprises any one or more, preferably at least 4, more preferably at least 6, even more preferably at least 8, most preferably at least 10, of the amino acids 1P/K, 3L/V, 6G/D, 7W/F, 8R/Q/K/E, 9R/K, 17G, 27Y/F/L, 30P, 32G, 44Y/F and 45L/I using the positional numbering of SEQ ID NO:1. These amino acids typically correspond to those at the same position in the wildtype MBD and are in some embodiments invariable.

In various other embodiments, the MBD core domain comprises any one or more, preferably at least 4, more preferably at least 6, even more preferably at least 8, most preferably at least 10, of the amino acids 1P/K, 3L/V, 6G/D, 7W/F, 8R/Q/K/E/T, 9R/K, 17G, 27Y/F/L, 30P, 32G, 44Y/F and 45L/I/F using the positional numbering of SEQ ID NO:1. These amino acids typically correspond to those at the same position in the wildtype MBD and are in some embodiments invariable.

In various other embodiments, the MBD core domain comprises any one or more, preferably at least 4, more preferably at least 6, even more preferably at least 8, most preferably at least 10, of the amino acids 1P/K/S/T/W/A/L/I/F/V, 3L/V/T/I, 6G/D/H, 7W/F, 8R/Q/K/E/T, 9R/K/M, 17G/A/Y/S/N/H, 27Y/F/L, 30P, 32G, 44Y/F/A and 45L/I/F/V, preferably any one or more of the amino acids 1P/K, 3L/V, 6G, 7W, 8K/E/T, 9R/K, 17G, 27Y/F/L, 30P, 32G, 44Y, 45L/F, using the positional numbering of SEQ ID NO:1. These amino acids typically correspond to those at the same position in the wildtype MBD and are in some embodiments invariable.

In various embodiments, the MBD core domain comprises any one or more, preferably at least 5, more preferably at least 10, even more preferably at least 15, most preferably at least 20, of the amino acids 1P/K, 2A/S/T, 3L/V, 4G/P, 5P/Q/C/E, 6G/D, 7W/F, 8R/Q/K/E, 9R/K, 10R/E/V/K, 11E/V/L, 12V/K, 13F/I/P/Q, 14R/K, 15K/R/L, 16F/S, 17G, 18A/L/K/R, 19T/S, 20C/A, 21G, 22R/K/H, 23S/R/F/Y, 24D/E, 25T/V, 26Y/F, 27Y/F/L, 28Q/F/Y/I, 29S/N/L, 30P, 31T/S/Q/D/A/H, 32G, 33D/K/L, 34R/K/A, 35I/F, 36R/K, 37S, 38K, 39V/P/S, 40E/Q/D, 41L, 42T/A/I, 43R/N/A, 44Y/F/V, 45L/I using the positional numbering of SEQ ID NO:1. These amino acids typically correspond to those at the same position in the wildtype MBD and are in some embodiments invariable.

In various embodiments, the MBD core domain comprises any one or more, preferably at least 5, more preferably at least 10, even more preferably at least 15, most preferably at least 20, of the amino acids 1P/K, 2A/S/T, 3L/V, 4G/P, 5P/Q/C/E, 6G/D, 7W/F, 8R/Q/K/E/T, 9R/K, 10R/E/V/K, 11E/V/L, 12V/K, 13F/I/P/Q, 14R/K, 15K/R/L, 16F/S, 17G, 18A/L/K/R, 19T/S, 20C/A, 21G, 22R/K/H, 23S/R/F/Y, 24D/E, 25T/V, 26Y/F, 27Y/F/L, 28Q/F/Y/I, 29S/N/L, 30P, 31T/S/Q/D/A/H, 32G, 33D/K/L, 34R/K/A, 35I/F, 36R/K, 37S, 38K, 39V/P/S, 40E/Q/D/S, 41L, 42T/A/I, 43R/N/A, 44Y/F/V, 45L/I/F using the positional numbering of SEQ ID NO:1. These amino acids typically correspond to those at the same position in the wildtype MBD and are in some embodiments invariable.

In various embodiments, the MBD core domain comprises any one or more, preferably at least 5, more preferably at least 10, even more preferably at least 15, most preferably at least 20, of the amino acids 1P/K/S/T/W/A/L/I/F/V, 2A/S/T/P/L/G/K/R, 3L,V/T/I, 4G/P/A/Q/E/L/K/R, 5P/Q/C/E/N/K/A/L/R/H/Y, 6G/D/H, 7W/F, 8R/Q/K/E/T, 9R/K/M, 10R/E/V/K/Q/S/M, 11E/V/L/N/T/H, 12V/K/S/A/I/C/R/G, 13F/I/P/Q/T/V/L/R/K, 14R/K/T, 15K/R/L/Q/S/N, 16F/S/L/T/I/D/G/V, 17G/A/Y/S/N/H, 18A/L/K/R/P/S, 19T/S/G/A/H/R, 20C/A/F/D/R/G/K/S, 21 G/I/V/W/L/M/S, 22R/K/H/Q/G/A/S, 23S/R/F/Y/T/G/V/M/L, 24D/E/H, 25T/V/I/A, 26Y/F/I/S/W/A/N, 27Y/F/L, 28Q/F/Y/I/R/K, 29S/N/H/A/T/G/L, 30P, 31T/S/Q/E/N/A/C/D/H, 32G, 33D/K/L/E/R, 34R/K/A/C/S/N, 35I/F/L/IM, 36R/E/S/K, 37S/T/Q/N, 38K/R/Y/F/M/V, 39V/P/S/R/I/N/Q/E/A, 40E/Q/S, 41L/V/I, 42T/A/I/M/V/E/F/Q, 43R/N/A/K/H, 44Y/F/A, 45L/I/F/V, preferably any one or more of the amino acids 1P/K, 2A/S/T, 3L/V, 4G/P, 5P/Q/C/E, 6G, 7W, 8K/E/T, 9R/K, 10R/E/V/K, 11E/V/L, 12V/K, 13F/I/P/Q, 14R, 15K/R/L, 16S/F, 17G, 18A/L/K/R, 19T/S, 20C/A, 21G, 22R/K/H, 23S/R/F/Y, 24D, 25T/V, 26Y/F, 27Y/F/L, 28Q/F/Y/I, 29S/N/L, 30P, 31T/S/Q/D/A/H, 32G, 33D/K/L, 34R/K/A, 35I/F, 36R, 37S, 38K, 39V/P/S, 40E/Q/S, 41L, 42T/A/I, 43R/N/A, 44Y, 45L/F, using the positional numbering of SEQ ID NO:1. These amino acids typically correspond to those at the same position in the wildtype MBD and are in some embodiments invariable.

In various embodiments, the isolated MBD variant comprises, in addition to the amino acid substitutions listed above, any one or more of the following substitutions using the positional numbering of SEQ ID NO:1: 29L, 31D/A/H, and 33E.

In various embodiments, the isolated MBD variant comprises one more additional amino acid sequences N- and/or C-terminal to the MBD core domain, each preferably 1-80 amino acids in length, more preferably the additional N-terminal amino acid sequence being 2 to 20 amino acids in length, and/or the additional C-terminal amino acid sequence being 10 to 60 amino acids in length. These additional amino acid sequences N- and/or C-terminal to the MBD core domain may correspond to the corresponding wildtype MBD as set forth in any one of SEQ ID Nos. 1-28 and may comprise 10 or less, preferably 6 or less, substitutions relative to the corresponding wildtype MBD as set forth in any one of SEQ ID Nos. 1-28.

If in any of the above embodiments amino acid positions have not been listed, it is, in various embodiments, preferred to retain the (natural) amino acids at these positions that have not been listed for substitution.

The isolated MBD variant has, relative to the corresponding wildtype MBD, an altered affinity for a DNA molecule comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

In various embodiments, said variant has an altered binding affinity, preferably increased affinity, for CpG dinucleotides and their complement in which
(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);
(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC);
(l) both cytosine bases are 5-carboxylated (caC);
(m) one cytosine base is 5-methylated cytosine (mC) and the other is non-modified (C); and/or
(n) both cytosine bases are 5-methylated (mC/mC).

In various embodiments, said variant has differential binding affinity for any two CpG dinucleotides and their complement selected from the following oxidized 5-methylated cytosine configurations:
(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);
(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC); and/or
(l) both cytosine bases are 5-carboxylated (caC).

Preferably, the isolated MBD variant comprises, consists essentially of or consists of any one of the amino acid sequences set forth in SEQ ID Nos. 46 to 67.

In a second aspect, the invention relates to a conjugate comprising the isolated MBD variant according to the invention.

Preferably, the conjugate further comprises an enzyme, preferably a nuclease or methyltransferase, or a detectable label, preferably a fluorophore.

In another aspect, the invention relates to a use of the isolated MBD variant as described herein or the conjugate as described herein for the determination of the methylation state of cytosine residues and/or oxidation state of 5-methylated cytosine residues in a CpG dinucleotide of interest and its complement in a DNA molecule or for the enrichment of DNA molecules comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

In still another aspect, the invention relates to a method for the determination of the methylation state of cytosine residues and/or oxidation state of the methyl group of 5-methylated cytosine residues in a CpG dinucleotide of interest and its complement in a DNA molecule, said method comprising
(a) providing a molecular probe comprising a Methyl-CpG binding domain (MBD) variant according to the invention that binds to the region of the DNA molecule comprising said CpG dinucleotide of interest and its complement and differentially binds to different methylated cytosine and/or oxidized 5-methyl-cytosine configurations in the CpG dinucleotide of interest and its complement, wherein said differential binding is detectable by differences in binding affinity;
(b) determining the methylation state of cytosine residues and/or oxidation state of said 5-methylated cytosine residues in said CpG dinucleotide of interest by contacting the molecular probe with the DNA molecule and determining the binding affinity of the molecular probe to the region of the DNA molecule comprising said CpG dinucleotide of interest.

In various embodiments of the method, the determination of the methylation state of cytosine residues and/or oxidation state of the methyl group of 5-methylated cytosine residues in a CpG dinucleotide of interest and its complement in a DNA molecule comprises determining the presence or the level of a nucleobase selected from the group consisting of cytosine (C), 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), in the CpG dinucleotide of interest and its complement.

In a further aspect, the invention relates to a method for the enrichment of DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), said method comprising
(a) providing a molecular probe comprising a Methyl-CpG binding domain (MBD) variant according to the invention that binds to the region of the DNA molecule comprising said CpG dinucleotide of interest and differentially binds to different methylated cytosine and/or oxidized 5-methylcytosine configurations in the CpG dinucleotide of interest and its complement, wherein said differential binding is facilitated by differences in binding affinity;
(b) contacting the molecular probe with a sample comprising DNA molecules comprising said CpG dinucleotide of interest and its complement under conditions that allow binding of the molecular probe to its target;
(c) enriching the DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), by separating the DNA molecules based on their affinity for the molecular probe.

In various embodiments of this method, the molecular probe is immobilized on a substrate.

Preferably, the molecular probe comprises an affinity ligand that allows immobilization of the molecular probe on a substrate.

The enrichment step of the method may comprise separating the complexes of the DNA molecules with the immobilized molecular probe from the non-complexed DNA molecules, the enrichment optionally including chromatography, centrifugation or magnetic bead separation.

In various embodiments of the methods according to the invention, the difference in binding affinity of the molecular probe is an increased binding affinity for an oxidized state of 5-methylated cytosine, in particular 5-hydroxymethylcytosine, relative to the non-oxidized 5-methylated cytosine.

In various embodiments of the methods according to the invention, the difference in binding affinity of the molecular probe is an increased binding affinity for a 5-methylated state of cytosine relative to the non-methylated cytosine.

The invention will be better understood with reference to the detailed description when considered in conjunction with the non-limiting examples and the accompanying figures.

### BRIEF DESCRIPTION OF THE DRAWINGS

FIG. 1**:** A) Chemical structures and color-code of the relevant nucleobases. B) Scheme of a (abridged subsequence of a DNA double-strand containing a CpG dinucleotide; the different modified cytosine species are indicated by color. C) Possible combinations of cytosine modifications at CpG dinucleotides.
**FIG. 2****:** A possible application of the invention is the isolation of short DNA duplexes ('fragments'), which can be derived e.g. from genomic DNA, that contain a CpG dinucleotide with a defined configuration of cytosine modifications. To this end, the MBD is immobilized on functionalized capture beads for affinity enrichment. The captured DNA fragments are eluted from the beads and can be sequenced by next-generation sequencing so that the genomic positions of the configuration states can be identified.
FIG. 3**:** A) Model of an MBD bound to a DNA double-strand containing a fully methylated CpG dinucleotide (mC/mC). The interaction with each DNA strand involves (but is not limited to) two distinct structures within the MBD, helix α₁ and loop L1; adapted from PDB:1ig4. B) Residues interacting with the nucleobases in proximity to the CpG dinucleotide; the numbering is according to the positional numbering of SEQ ID NO:1; bold sites indicate amino acids, which can be preferably substituted relative to the wildtype sequence.

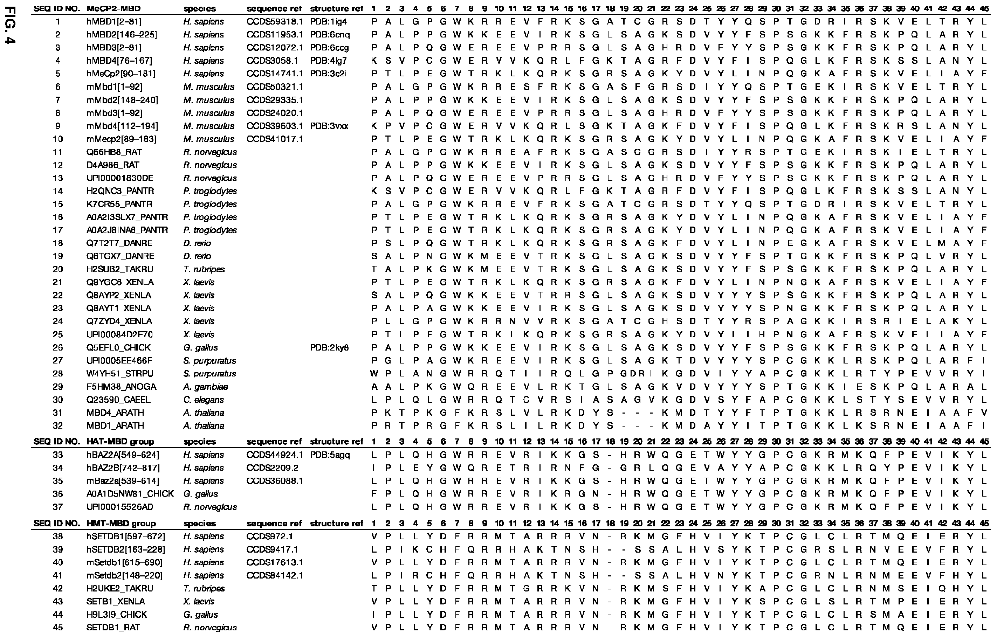

**FIG. 4****:** Overview of amino acid sequences (MBD core domain) of SEQ ID Nos. 1-45.

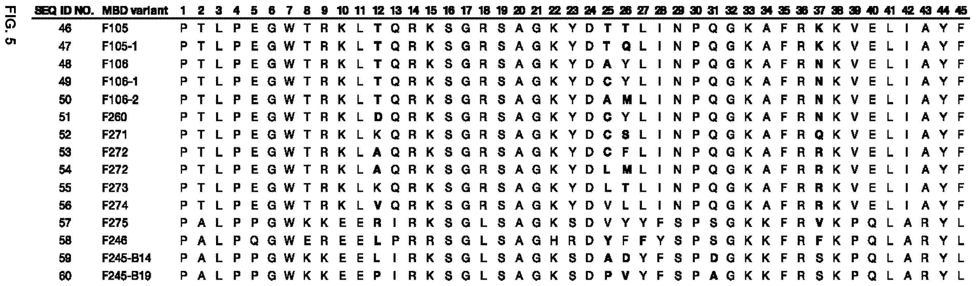

**FIG. 5****:** Overview of amino acid sequences (MBD core domain) of SEQ ID Nos. 46-60 (bold sites indicate substituted amino acids relative to the corresponding wildtype sequence).

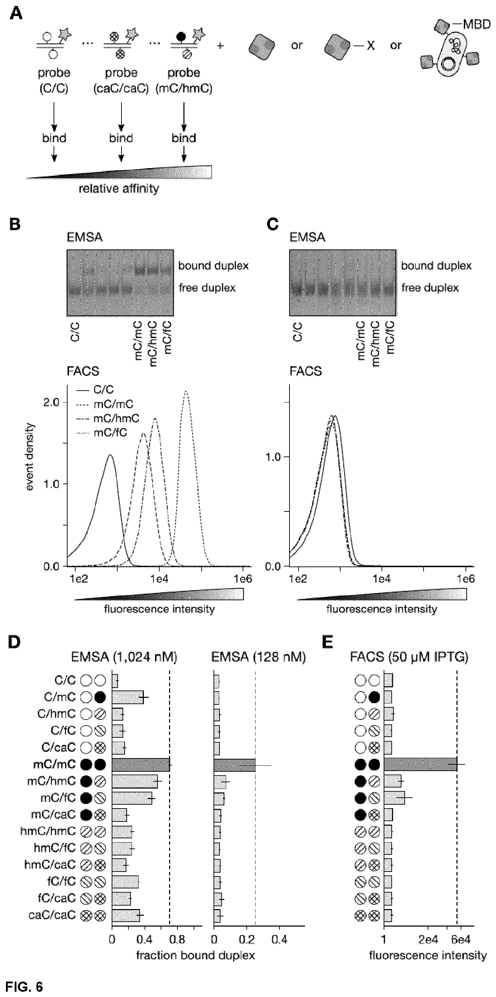

FIG. 6**:** A) Probe-wise determination of binding affinity using fluorescently labelled DNA duplexes that contain a single CpG dinucleotide with one of the configurations of FIG. 1; these are incubated with an isolated and purified MBD protein or an isolated and purified MBD protein fused to a 'tag' (as specified herein) or to an isolated population of different *E. coli* clones that each present a single species of MBD protein on their cell surface. Typically, relative affinity of isolated and purified MBD proteins is determined with an electrophoretic mobility shift assay (EMSA), whereas the relative affinity of MBD proteins displayed on the cell surface is determined using a fluorescence-activated cell sorting (FACS) on a flow cytometer. B) Assay validation for an mC/mC-binding wildtype MBD protein containing SEQ ID NO:2 (hMBD2[148-225] on EMSA and FACS (not all probes shown)). C) Assay validation for a non-binding wildtype MBD protein containing SEQ ID NO:3 (hMBD3[2-81]) on EMSA and FACS (not all probes shown). D) Systematic profiling of a wildtype MBD protein containing SEQ ID NO:2 (hMBD2[148-225]) at a high and a low protein concentration on EMSA. E) The same MBD protein displayed on a cell surface after induction of MBD expression with 50 µM isopropyl β-D-1-thiogalactopyranoside for 60 min. Population mean fluorescence intensities of three independent replicates are shown.

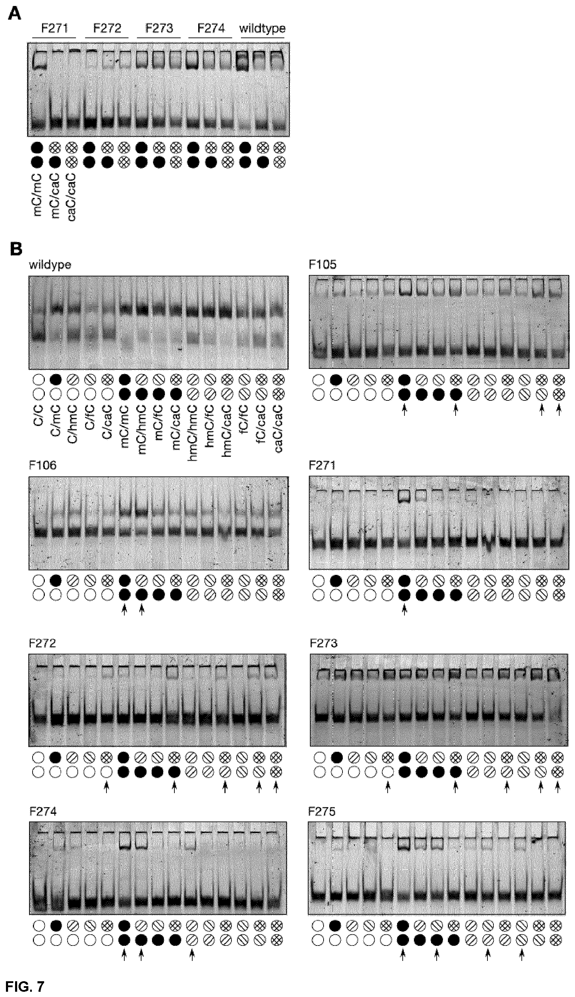

FIG. 7**:** A) Selectivity of the hMeCp2[90-181]-derived purified MBD proteins F271, F272, F273, and F274 ('wildtype'= hMeCp2[90-181]; SEQ ID NO:69) on EMSA using mC/mC, mC/caC and caC/caC probes. B) Full EMSA-profile of the purified proteins F105, F106, F271, F272, F273, F274 and the hMBD2[148-225]-derived protein F275 on EMSA. Arrows indicate distinct differences from wildtype behavior. F105, F271-F274 were expressed as MBP-fusion protein. The MBD core domain of the purified proteins F105, F106, F271, F272, F273, F274, F275 has the amino acid sequence set forth in SEQ ID Nos. 46, 48, 52, 53, 55, 56 and 57, respectively.

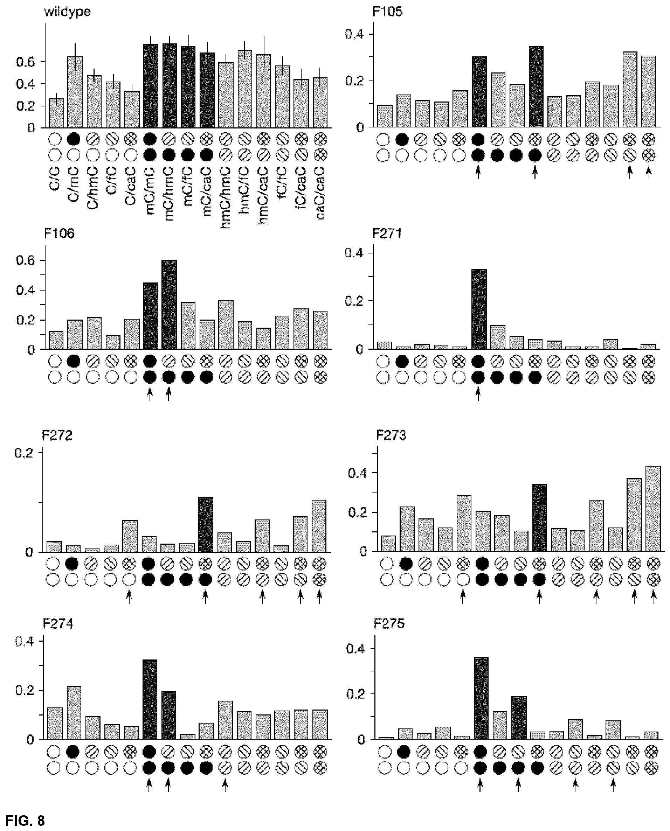

**FIG. 8****:** Quantification of the selectivity based on EMSA as shown in FIG. 7B as the fraction of bound DNA duplex at 1,024 nM MBD protein. The main selectivity within the group of mC/mC, mC/hmC, mC/fC, and mC/caC are indicated by shading. Arrows indicate distinct differences from wildtype behavior.

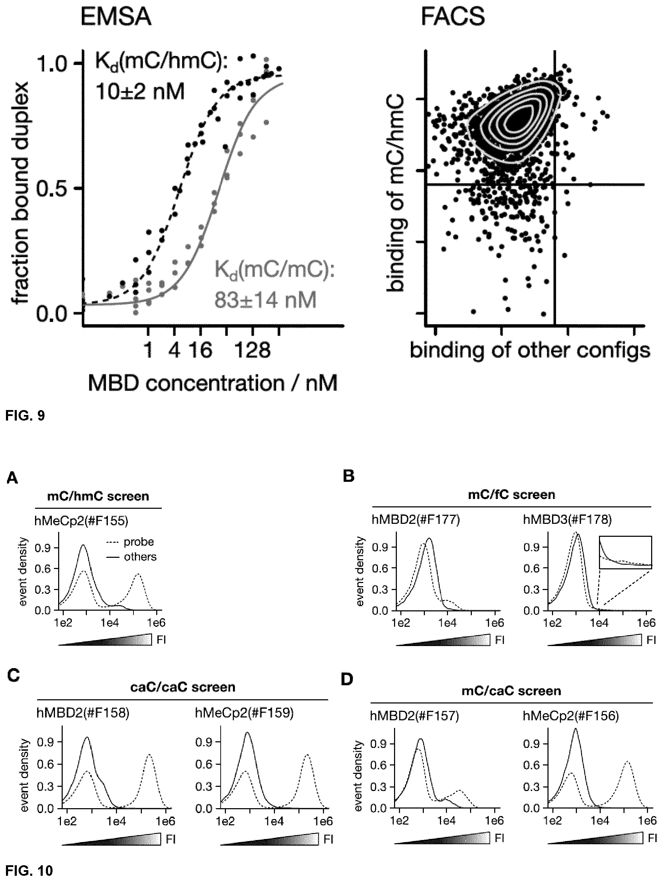

**FIG. 9****:** Determination of the dissociation constant Kd for the hMeCp2 [90-181]-derived ('wildtype') purified MBD protein F106 (MBD core domain sequence SEQ ID NO:48; 'wildtype'= hMeCp2[90-181]; SEQ ID NO:69) for mC/hmC and mC/mC using EMSA; and validation of binding selectivity in a FACS assay with surface-displayed F106 and differentially labeled DNA duplexes at equimolar ratio.
**FIG. 10****:** Unpurified candidates (mixtures of surface-displayed MBD domains) for selective recognition of the oxidized mC CpG configurations as indicated. The candidates are derived from codon-degenerated scaffolds comprising SEQ ID Nos. 2, 3, or 5. Relative selectivity was quantified in separate reactions with the same fluorophore-streptavidin conjugate using either a dsDNA probe containing the indicated configuration or an equimolar mixture of all other probes.

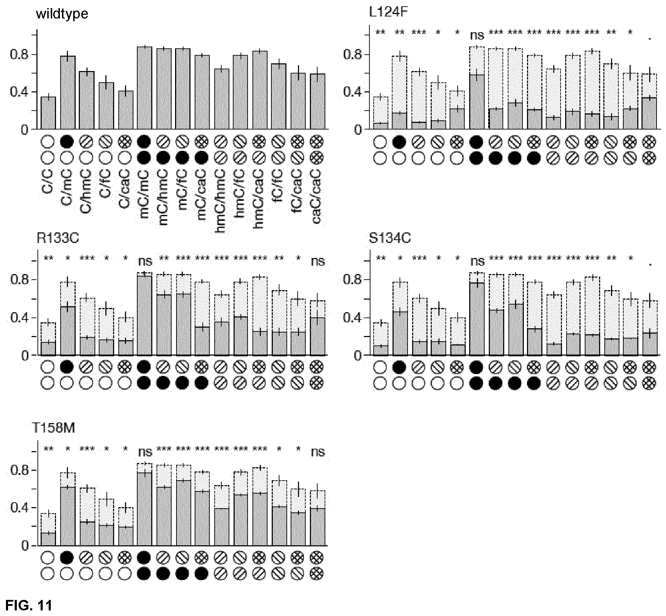

**FIG. 11****:** Representative single measurements of the fraction of bound labelled DNA duplex are summarized (mean) as bar graphs at high MBD protein concentration. Error bars indicate standard error of the mean with two-sided Student's t-test against 'wildtype' MeCp2[90-181] (SEQ ID NO:69) (ns: p in (0.1, 1], . (0.05, 0.1], * (0.01, 0.05], ** (0.001, 0.01], *** [0, 0.001]) for the variants L124F (SEQ ID NO:73), R133C (SEQ ID NO:74), S134C (SEQ ID NO:75) and T158M (SEQ ID NO:76) of human MeCp2[90-181] (SEQ ID NO:69).

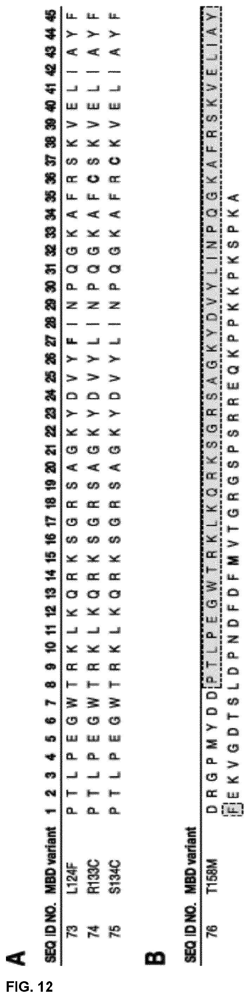

FIG. 12**:** A) Overview of amino acid sequences (MBD core domain) of SEQ ID Nos. 73 to 75; B) T158M variant of human MeCp2[90-181] (SEQ ID NO:76) (grey-shaded amino acids indicate the MBD core domain and bold amino acids indicate the amino acid substitution).

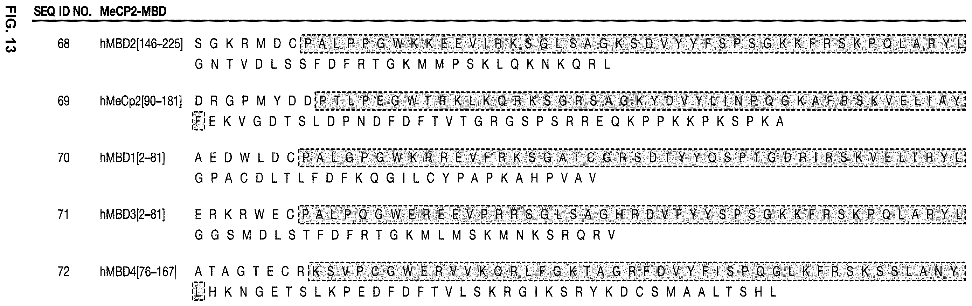

**FIG. 13****:** Wildtype MBD protein sequences of human MBD2[146-225] (SEQ ID NO:68), human MeCp2[90-181] (SEQ ID NO:69), human MBD1[2-81] (SEQ ID NO:70), human MBD3[2-81] (SEQ ID NO:71), human MBD4[76-167] (SEQ ID NO:72) (grey-shaded amino acids indicate the MBD core domain).

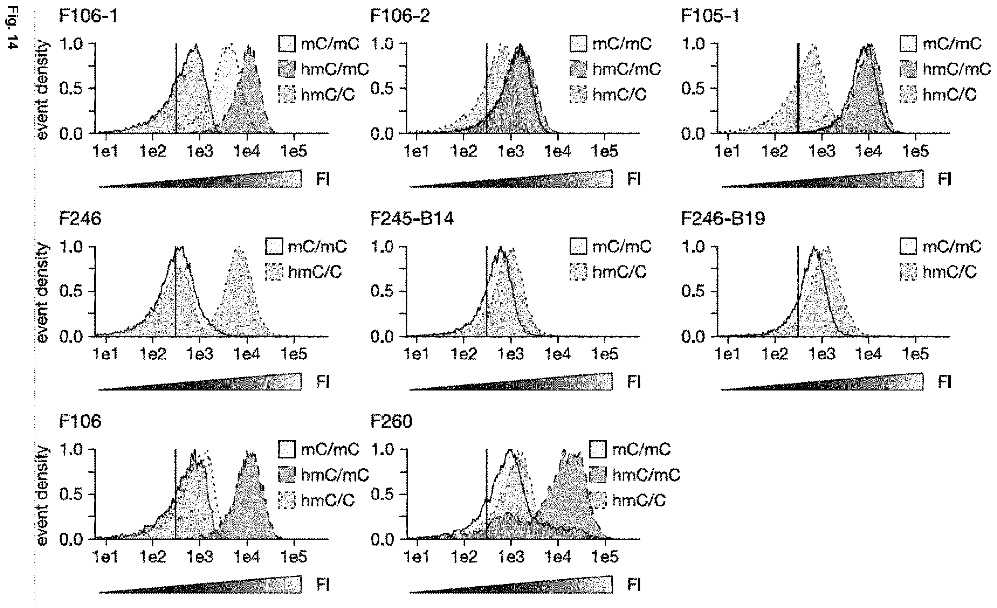

**FIG. 14****:** Quantification of selectivity based on FACS for purified surface-displayed MBD domains of F105-1, F106-1, F106-2, F260, F246, F245-B14, and F246-B19 (SEQ ID Nos. 47, 49, 50, 51, 58, 59, 60).

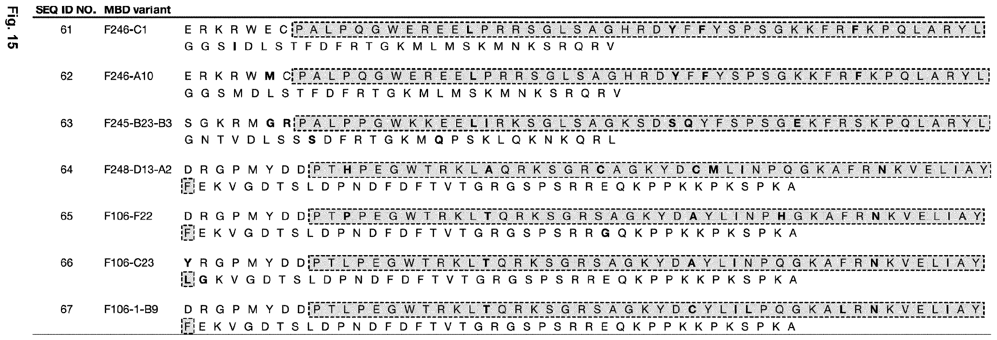

**FIG. 15****:** Overview of amino acid sequences of SEQ ID Nos. 61-67 (grey-shaded amino acids indicate the MBD core domain and bold amino acids indicate the amino acid substitution).

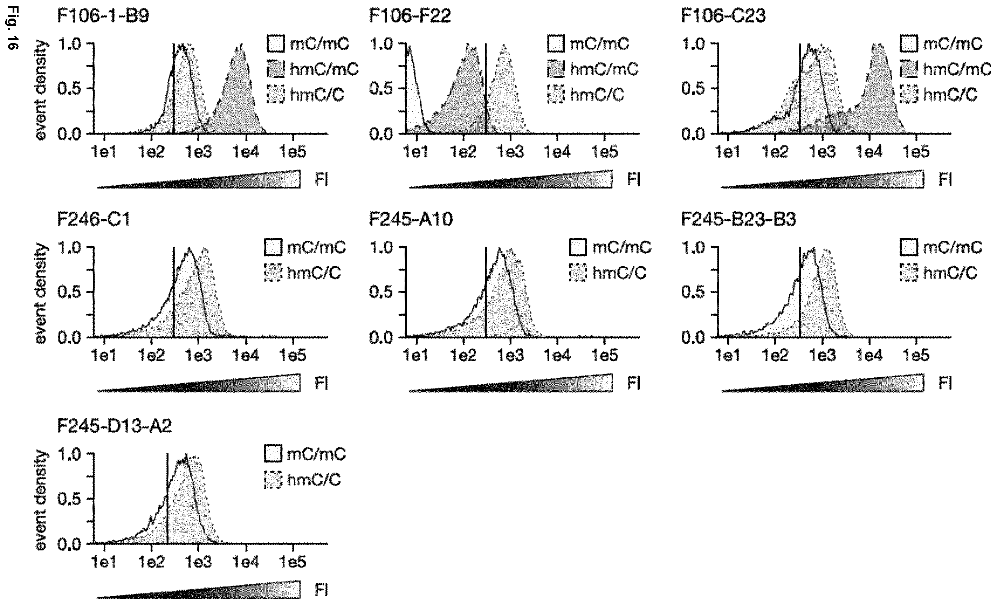

**FIG. 16****:** Quantification of selectivity based on FACS of purified surface-displayed MBD domains of F106-1-B9, F106-F22, F106-C23, F246-C1, F245-A10, F245-B23-B3, and F245-D13-A2 (SEQ ID Nos. 61-67).

### DETAILED DESCRIPTION OF THE INVENTION

The terms "one or more" or "at least one", as interchangeably used herein, relate to at least 1, or at least 2, 3, 4, 5, 6, 7, 8, 9, 10, 11, 12, 13, 14, 15, 20, 25 or a plurality of species, e.g. at least one amino acid substitution. In this connection, the term "plurality" means more than one, preferably 2 or more, such as up to 1000.

Numeric values specified without decimal places here refer to the full value specified with one decimal place, i.e. for example, 99 % means 99.0 %, unless otherwise defined.

The terms "approx." and "about", in connection with a numerical value, refer to a variance of ±10 %, preferably ±5 %, more preferably ±2 %, more preferably ±1 %, more preferably ±0.1 %, and most preferably less than ±0.1 %, with respect to the given numerical value.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

"Substantially", for example in "substantially purified", typically means that the given amount or property is dominant compared to other amounts or properties. With respect to purification, this means that little amounts of impurities, i.e. the to-be-separated species, can still be present. Preferably, these impurities are comprised in amounts of less than 10 wt.-%, more preferably less than 5 wt.-%, more preferably less than 1 wt.-%, more preferably less than 0.1 wt. %, more preferably less than 0.01 wt.-%, more preferably less than 0.001 wt.-%, most preferably these impurities are not present, if not explicitly stated otherwise. With respect to properties, it means that the original properties/functionalities are retained to a considerable extent, for example by at least 80% with respect to functionalities and activities.

The invention is defined by the appended claims.

Described herein is an isolated Methyl-CpG binding domain (MBD) variant, comprising an MBD core domain that has at least 60 %, preferably at least 70 %, sequence homology or sequence identity relative to any one of SEQ ID Nos.1-45 and comprising at least one amino acid substitution relative to the corresponding wildtype MBD in at least one of the positions corresponding to positions 12, 14, 19, 21, 22, 23, 24, 25, 26, 27, 29, 31, 33, 35, 36, 37, 38, 39, 40 and 45, for example
12, 14, 19, 21, 22, 23, 24, 25, 26, 27, 35, 36, 37, 38, 39 and 40; or
12, 19, 21, 25, 26, 27, 29, 31, 33, 35, 36, 37, 38, 39, 40 and 45; or
12, 19, 21, 22, 23, 25, 26, 27, 35, 36, 37, 38, and 39; or
12, 19, 21, 25, 26, 27, 29, 31, 33, 35, 36, 37, 38 and 45; or
12, 25, 26, 27, 29, 31, 33, 35, 37 and 45; or
12, 25, 26, 27, 36 and 37; or
12, 25, 26, 37, in SEQ ID NO:1.

While the positional numbering is given above based on SEQ ID NO:1, the positional numbering could be similarly given relative to any one of SEQ ID Nos. 2-28, in particular 1-5, as these sequences share sufficient homology to each other that positional numbering would be identical. Accordingly, any positional numbering indicated herein to be based on SEQ ID NO:1 could similarly be done based on any one of SEQ ID Nos. 2-28, preferably 2-5.

In various embodiments of the present disclosure, the isolated Methyl-CpG binding domain (MBD) variant comprises an MBD core domain that has at least 60 %, preferably at least 70 %, sequence homology or identity relative to any one of SEQ ID Nos.1-5, preferably SEQ ID NO:2 or 5, and comprises at least one amino acid substitution relative to the corresponding wildtype MBD in at least one of the positions corresponding to positions
12, 14, 19, 21, 22, 23, 24, 25, 26, 27, 29, 31, 33, 35, 36, 37, 38, 39, 40 and 45, for example
12, 14, 19, 21, 22, 23, 24, 25, 26, 27, 35, 36, 37, 38, 39 and 40; or
12, 19, 21, 25, 26, 27, 29, 31, 33, 35, 36, 37, 38, 39, 40 and 45; or
12, 19, 21, 22, 23, 25, 26, 27, 35, 36, 37, 38, and 39; or
12, 19, 21, 25, 26, 27, 29, 31, 33, 35, 36, 37, 38 and 45; or
12, 25, 26, 27, 29, 31, 33, 35, 37 and 45; or
12, 25, 26, 27, 36 and 37; or
12, 25, 26, 37, in SEQ ID NO:1.

As used herein, the term "Methyl-CpG binding domain (MBD) variant" refers to a Methyl-CpG binding domain or a segment or fragment thereof that varies in its sequence compared to a naturally occuring version of the same molecule. In various embodiments, this means that its sequence has one or more amino acids added, deleted, substituted or otherwise chemically modified in comparison to the corresponding wildtype MBD or the corresponding segment or fragment thereof. It is however understood that such variants substantially retain the same properties as the wildtype MBD to which they are compared, in particular such that they still have the characteristic functionality and activity of an MBD.

Typically, the modified MBD (variant) is "isolated" from the cell in which it was generated, using standard techniques known to the person skilled in the art. The term can also apply to a variant, which has been substantially purified from other components, which naturally accompany the variant, e.g., proteins, RNA or DNA which naturally accompany it in the cell.

However, it is also included in the scope of the present invention that the variant can be displayed on the surface of a cell, preferably on the surface of E. *coli,* using standard techniques known to the person skilled in the art (e.g. AIDA surface display). The term therefore includes, for example, a recombinant MBD or a segment or fragment thereof, which is encoded by a nucleic acid incorporated into a vector, into an autonomously replicating plasmid or virus, or into the genomic DNA of a prokaryote or eukaryote, or which exists as a separate molecule (e.g., as a cDNA or a genomic or cDNA fragment produced by PCR or restriction enzyme digestion) independent of other sequences.

In some embodiments, the variant is based on the Methyl-CpG binding domain of an MBD or MeCp2 protein, which is recombinantly produced, preferably in E. *coli,* and subsequently either isolated from the cell for further use or displayed on the cell surface, preferably on the cell surface of E. *coli.*

The term "MBD core domain" refers to a domain comprised in the amino acid sequence of the MBD wildtype or variant, which is typically necessary to enable the binding of MBD with a DNA molecule comprising a CpG dinucleotide, if not explicitly stated otherwise. The core domain is typically about 45 amino acids in length. In various embodiments, the core domain is 42 to 46 amino acids in length, preferably 42, 43, 44, 45 or 46 amino acids in length.

Preferably, said MBD variant has relative to the wildtype MBD an altered affinity, preferably an increased or decreased affinity, for a DNA molecule comprising a CpG dinucleotide of interest and its complement in which at least one cytosine nucleobase in the CpG dinucleotide of interest is modified, wherein the cytosine nucleobase is selected from the group of cytosine (C), 5-methylcytosine (mC), 5-hydroxymethylcytosine (hmC), 5-formylcytosine (fC), and 5-carboxylcytosine (caC), preferably the cytosine nucleobase in the CpG dinucleotide of interest is modified and is selected from the group of 5-methylcytosine (mC), 5-hydroxymethylcytosine (hmC), 5-formylcytosine (fC), and 5-carboxylcytosine (caC), more preferably from the group of 5-hydroxymethylcytosine (hmC), 5-formylcytosine (fC), and 5-carboxylcytosine (caC).

"Altered affinity", as used herein, means that the affinity if tested in a suitable assay, such as calorimetry, surface plasmon resonance, etc, detectably differs compared to the corresponding wildtype protein. "Detectably differs" thus means that the difference falls outside the error margins of the measurement. In various embodiments, such an alteration or difference in affinity means a change by at least 10 %, at least 20 %, at least 50 % or more. In various embodiments, the change is at least 2-fold, more preferably at least 5-fold or most preferably at least one order of magnitude (10-fold). This applies in two directions, i.e. the affinity may be double the original value or half the original value, etc.

The term "CpG-dinucleotide" describes a part of a DNA molecule in which two nucleotides containing the nucleobases cytosine and guanine are linked to each other. Typically, the CpG-dinucleotide contains or consists of the unit deoxycytidine-phosphate-deoxyguanosine (in 5'-3' direction). In general, without being limited to this, the presence and level of the CpG-dinucleotide in a DNA molecule is organism- and region-dependent. CpG sites occur with high frequency in genomic regions called CpG islands (or CG islands) with the cytosine often being subject to methylation. In the target nucleic acid sequences, i.e. those sequences targeted by the MBD, the nucleobase cytosine can thus be modified and selected from the group of 5-methylcytosine (5mC), 5-hydroxymethylcytosine (hmC), 5-formylcytosine (fC), and 5-carboxylcytosine (caC). It is an object of the invention to design MBD variants that can distinguish between those different variants.

The term "sequence identity" as used herein refers to polypeptides, peptides or proteins or segments or fragments thereof that share identical amino acids at corresponding positions or nucleic acids sharing identical nucleotides at corresponding positions. The broader concept of "sequence homology" also takes conserved amino acid exchanges into account in the case of amino acid sequences, i.e. amino acids having similar chemical activity, since they usually perform similar chemical activities within the protein. Under the concept of sequence homology, conservative amino acid changes are thus not counted as changes. Conservative replacements means a change within the following groups of amino acids:
Hydrophobic: M, A, V, L, I
Neutral/Hydrophilic: S, C, T, N, Q
Acidic: D, E
Basic: H, K, R
Residues that influence chain orientation: G, P
Aromatic: W, Y, F

The determination of percent sequence homology or percent sequence identity described herein between two amino acid or nucleotide sequences can be accomplished using a mathematical algorithm. For example, a mathematical algorithm useful for comparing two sequences is the algorithm incorporated into the BLASTN and BLASTX programs and can be accessed, for example, at the National Center for Biotechnology Information (NCBI) world wide web site having the universal resource locator "www.ncbi.nlm.nih.gov/BLAST". Blast nucleotide searches can be performed with BLASTN program, whereas BLAST protein searches can be performed with BLASTX program or the NCBI "blastp" program.

Identity or homology information can be provided regarding whole polypeptides, peptides, proteins or genes or only regarding individual regions. However, identity or homology information in the present application relates to the entire length of the particular nucleic acid or amino acid sequence indicated. For example, percent homology is based on the entire amino acid sequence of the MBD core domain according to any one of SEQ ID Nos. 1-45, preferably to any one of SEQ ID Nos. 1-28, more preferably to any one of SEQ ID Nos. 1-10, in particular according to any one of SEQ ID NO: 1, 2, 3, 4, 5, 6, 7, 8, 9, or 10, most preferably to any one of SEQ ID Nos. 1-5.

According to the present invention, the feature "at least 60 % sequence homology" includes every variation, in particular every amino acid substitution, in the MBD core domain of the Methyl-CpG-binding domain variant which is/are present relative to any one of SEQ ID Nos. 1-45, preferably to any one of SEQ ID Nos. 1-28, more preferably to any one of SEQ ID Nos. 1-10, most preferably to any one of SEQ ID Nos. 1-5, unless indicated otherwise. "60% homology" thus means that 60% of all amino acids present at the corresponding positions in the variant are either substituted such that they are still homologous or are unaltered. In preferred embodiments, the MBD core domain of the MBD variant has at least 65, 70, 75 or 80 % sequence homology or sequence identity to any one of SEQ ID Nos.1-45, preferably to any one of SEQ ID Nos.1-28, more preferably to any one of SEQ ID NO:1, 2, 3, 4, 5, 6, 7, 8, 9, or 10, unless indicated otherwise. In various embodiments, the "60% homology" are "60% identity".

In various embodiments, the sequence homology or identity is at least 85, 86, 87, 88, 89, 90, 91, 92, 93, 94, 95, 96, or 97 %. In various embodiments, sequence identity is preferred over sequence homology.

The variants comprise at least one amino acid substitution relative to the corresponding wildtype MBD in at least one of the positions corresponding to positions
12, 14, 19, 21, 22, 23, 24, 25, 26, 27, 29, 31, 33, 35, 36, 37, 38, 39, 40 and 45, for example
12, 14, 19, 21, 22, 23, 24, 25, 26, 27, 35, 36, 37, 38, 39 and 40; or
12, 19, 21, 25, 26, 27, 29, 31, 33, 35, 36, 37, 38, 39, 40 and 45; or
12, 19, 21, 22, 23, 25, 26, 27, 35, 36, 37, 38, and 39; or
12, 19, 21, 25, 26, 27, 29, 31, 33, 35, 36, 37, 38 and 45; or
12, 25, 26, 27, 29, 31, 33, 35, 37 and 45; or
12, 25, 26, 27, 36 and 37; or
12, 25, 26, 37, or, if SEQ ID NO:3 is used as a reference, 12, 25, 27, 37 according to SEQ ID NO:1. SEQ ID NO:1 is used as a reference here to allow positional numbering.

The corresponding positions to the positions in SEQ ID NO:1 can be determined by an alignment, as explained above. In various embodiments, the variants described herein comprise only 1 amino acid substitution relative to the template sequence. In various other embodiments, the variants comprise two or more, 3 or more, 4 or more, 5 or more, 6 or more, 7 or more, 8 or more, 9 or more, 10 or more, 11 or more, 12 or more, 13 or more, 14 or more, 15 or more, 16 or more, 17, or more, 18 or more, 19 or more or 20 substitutions. These substitutions are preferably selected from substitutions in the positions listed above, with the target amino acids being any amino acid with the exception of the amino acid occurring naturally at this position, preferably being those described herein. Accordingly, if the natural amino acid in the position corresponding to position 12 is already V, a polypeptide having such an amino acid would not be considered a "substitution" in the sense of the present invention. It is understood that if a given reference sequence already contains the given substitution in its wildtype sequence, another substitution that does exchange a naturally occurring amino acid to a non-naturally occurring amino acid needs to be present. Said other substitution then needs to be also selected from the given list of possible substitutions. This concerns the following substitutions that occur as natural amino acids in the listed reference sequences:
12V in SEQ ID Nos. 1-3, 7, 8, 12, 13, 15, 19, 20, 22-24, 26-27, 29 and 31;
12S in SEQ ID NO:6;
12A in SEQ ID Nos. 38-45;
12R in SEQ ID Nos. 33-37;
25I in SEQ ID NO: 6;
25T in SEQ ID Nos. 1, 15, 24, 31 and 33-37;
25A in SEQ ID NO:32;
26S in SEQ ID Nos. 30 and 39;
26F in SEQ ID Nos. 3 and 8;
27F in SEQ ID NO: 4;
35L in SEQ ID Nos. 27, 28, 30-32, 34, and 38-45;
37N in SEQ ID Nos. 39, 41, and 42;
37Q in SEQ ID Nos. 33-37.

In various embodiments, if any of the above substitutions occurs in the variants of the invention, the reference sequence is not any of the reference sequences listed above in which it naturally occurs. It is however possible that one of these amino acids is present in these sequences together with a "real" substitution that exchanges a wildtype amino acid for a non-wildtype amino acid.

In various preferred embodiments, said at least one amino acid substitution relative to the corresponding wildtype MBD using the positional numbering of SEQ ID NO:1 is selected from
(1) 12V, 12S, 12T, 12A, 12R, 12D, 12L, 12P, 25I, 25T, 25A, 25C, 25L, 25Y, 25P, 25S, 26T, 26S, 26F, 26L, 26D, 26V, 26Q, 26M, 27F, 35L, 36C, 37N, 37K, 37Q, 37R, 37V and 37F;
(2) 12V, 12S, 12T, 12A, 12R, 12D, 12L, 12P, 25I, 25T, 25A, 25C, 25L, 25Y, 25P, 25S, 26T, 26S, 26F, 26L, 26D, 26V, 26Q, 26M, 37N, 37K, 37Q, 37R, 37V and 37F;
(3) 12V, 12S, 12T, 12A, 12R, 25I, 25T, 25A, 25C, 25L, 26T, 26S, 26F, 26L, 26Q, 37N, 37K, 37Q, 37R and 37V;
(4) 12V, 12S, 12T, 25I, 25T, 25A, 25C, 26T, 26S, 26L, 26Q, 37N, 37R, and 37K.

In various embodiments, said at least one amino acid substitution relative to the corresponding wildtype MBD using the positional numbering of SEQ ID NO:1 is selected from 12S, 12T, 12A, 12V, 12R, 25I, 25T, 25A, 25C, 25L, 26T, 26S, 26F, 26L, 27F, 36C, 37N, 37K, 37Q, 37R, 37V and 37C, preferably 12S, 12T, 12A, 12V, 12R, 25I, 25T, 25A, 25C, 25L, 26T, 26S, 26F, 26L, 37N, 37K, 37Q, 37R and 37V, more preferably 12S, 12T, 25I, 25T, 25A, 26T, 37N and 37K.

In various embodiments said at least one amino acid substitution relative to the corresponding wildtype MBD using the positional numbering of SEQ ID NO:1 is selected from 12V, 12S, 12T, 12A, 12R, 12D, 12L, 12P, 25I, 25T, 25A, 25C, 25L, 25Y, 25P, 25S, 26T, 26S, 26F, 26L, 26D, 26V, 26Q, 26M, 27F, 29L, 31A, 31D, 31H, 33E, 35L, 36C, 37N, 37K, 37Q, 37R 37V, 37F, and 45L, preferably 12V, 12S, 12T, 12A, 12R, 12D, 12L, 12P, 25I, 25T, 25A, 25C, 25L, 25Y, 25P, 25S, 26T, 26S, 26F, 26L, 26D, 26V, 26Q, 26M, 27F, 29L, 31A, 31D, 31H, 33E, 37N, 37K, 37Q, 37R 37V, 37F, and 45L, more preferably 12V, 12T, 12A, 12R, 12D, 12L, 12P, 25T, 25A, 25C, 25L, 25Y, 25P, 26T, 26S, 26F, 26L, 26D, 26V, 26Q, 26M, 27F, 29L, 31A, 31D, 31H, 33E, 35L, 36C, 37N, 37K, 37Q, 37R 37V, 37F, and 45Lusing the positional numbering of SEQ ID NO:1.

The term "positional numbering" refers to the numbering and arrangement of the present amino acids of the MBD core domain of the MBD wildtype construct according to SEQ ID Nos. 1, if not explicitly stated otherwise. SEQ ID Nos. 2-27 and 29-30 have the same positional numbering as SEQ ID NO:1. In contrast, SEQ ID NO: 28 comprises an insertion at position 20 and SEQ ID Nos. 31-45 contain amino acid deletions (FIG. 4). "Positions corresponding to positions [...] in SEQ ID NO:1" thus refer to those positions that correspond to the respective numbered position in SEQ ID NO:1 in an alignment.

In various embodiments, the isolated MBD variant comprises at least two, preferably at least three, amino acid substitutions selected from 12V, 12S, 12T, 12A, 12R, 12D, 12L, 12P, 25I, 25T, 25A, 25C, 25L, 25Y, 25P, 25S, 26T, 26S, 26F, 26L, 26D, 26V, 26Q, 26M, 27F, 29L, 31A, 31D, 31H, 33E, 35L, 36C, 37N, 37K, 37Q, 37R 37V, 37F, and 45L.

In various embodiments, the isolated MBD variant comprises at least one, preferably at least two, amino acid substitution selected from 12V, 12S, 12T, 12A, 12R, 12D, 12L, 12P, 25I, 25T, 25A, 25C, 25L, 25Y, 25P, 25S, 26T, 26S, 26F, 26L, 26D, 26V, 26Q, 26M, 27F, 29L, 31A, 31D, 31H, 33E, 35L, 36C, 37N, 37K, 37Q, 37R 37V, 37F, and 45L using the positional numbering of SEQ ID NO:1; and
at least one amino acid substitution in at least one of the positions corresponding to positions 19, 21, 38 and 40 in SEQ ID NO:1.

In various embodiments, the isolated MBD variant comprises at least one, preferably at least two, amino acid substitution selected from 12V, 12S, 12A, 12R, 25I, 25T, 25A, 26S, 26F, 27F, 35L, 37N, and 37Q using the positional numbering of SEQ ID NO:1; and

at least one amino acid substitution selected from 12T, 12D, 12L, 12P, 25C, 25L, 25Y, 25P, 25S, 26T, 26L, 26D, 26V, 26Q, 26M, 29L, 31A, 31D, 31H, 33E, 36C, 37K, 37R 37V, 37F, and 45L using the positional numbering of SEQ ID NO:1.

For example, the amino acid sequence set forth in SEQ ID NO:2, can be modified with at least one of the following amino acid substitutions: V12S, V12T, V12R, V25I, V25T, V25A, Y26T, S37N, S37K, S37V. For example, "V12S" means that the amino acid valine which is present at position 12 in the wildtype MBD core domain of the human MBD2 is substituted by (replaced with) a serine.

In a preferred embodiment, the amino acid sequence set forth in SEQ ID NO:2 is modified with at least the following amino acid substitutions: V12R and S37V. These may be the only amino acid substitutions present.

In another preferred embodiment, the amino acid sequence set forth in SEQ ID NO:5 is modified with one of the following amino acid substitution(s): i) L27F; ii) R36C, iii) S37C, iv) K12T, V25T, Y26T, S37K; iv) K12T, V25A, S37N; v) K25C, Y26S, S37Q; vi) K12A, V25C, Y26F, S37R; vii) K25L, Y26T, S37R; viii) K12V, Y26L, S37R.

In various embodiments, the isolated MBD variant comprises one of the following amino acid substitutions or amino acid substitution combinations, preferably in its MBD core domain:
i) 12S; ii) 12T; iii) 25I; iv) 25T; v) 26T; vi) 37N; vii) 37K; viii) 12S, 25I; ix) 12S, 25T; x) 12S, 26T; xi)12S, 37N; xii) 12S, 37K; xiii) 12T, 25I; xiv) 12T, 25T; xv) 12T, 26T; xvi) 12T, 37N; xvii) 12T, 37K; xviii) 25I, 26T; xix) 25I, 37N; xx) 25I, 37K; xxi) 25T, 26T; xxii) 25T, 37N; xxiii) 25T, 37K; xxiv) 26T, 37N; xxv) 26T, 37K; xxvi) 12S, 25I, 26T; xxvii) 12S, 25I, 37N; xxviii) 12S, 25I, 37K; xxix) 12S, 25T, 26T; xxx) 12S, 25T, 37N; xxxi) 12S, 25T, 37K; xxxii) 12S, 26T, 37N; xxxiii) 12S, 26T, 37K; xxxiv) 12T, 25I, 26T; xxxv) 12T, 25I, 37N; xxxvi) 12T, 25I, 37K; xxxvii) 12T, 25T, 26T; xxxviii) 12T, 25T, 37N, xxxix) 12T, 25T, 37K; xl) 12T, 26T, 37N; xli) 12T, 26T, 37K; xlii) 25I, 26T, 37N; xliii) 25I, 26T, 37 K; xliv) 25T, 26T, 37N; xlv) 25T, 26T, 37K; xlvi) 12S, 25I, 26T, 37N; xlvii) 12S, 25I, 26T, 37K; xlviii) 12S, 25T, 26T, 37N; xlix) 12S, 25T, 26T, 37K; I) 12T, 25I, 26T, 37N; li) 12T, 25I, 26T, 37K; lii) 12T, 25T, 26T, 37N; or liii) 12T, 25T, 26T, 37K, preferably relative to the corresponding wildtype MBD according to any one of SEQ ID Nos. 1-28, wherein the positional numbering is according to SEQ ID NO:1.

In various embodiments, the isolated MBD variant comprises one of the following amino acid substitutions or amino acid substitution combinations, preferably in its MBD core domain:
i) 12T, 25T, 26T, 37K; ii) 12T, 25A, 37N; iii) 25C, 26S, 37Q; iv) 12A, 25C, 26F, 37R; v) 25L, 26T, 37R; vi) 12V, 26L, 37R; vii) 12R, 37V, viii) 27F; ix) 36C; x) 37C; relative to the corresponding wildtype MBD according to any one of SEQ ID Nos. 1-28, preferably to any one of SEQ ID Nos. 1, 2, 3, 4 or 5, more preferably to any one of SEQ ID NO:2, 3 or 5 or 2 or 5, wherein the positional numbering is according to SEQ ID NO:1.

In various embodiments, the variant comprises any two or more, preferably three or all, of the substitutions set forth in the following sets of substitutions:
(1) 12T, 25T, 26T, 37K;
(2) 12T, 25A, 37N;
(3) 25C, 26S, 37Q;
(4) 12A, 25C, 26F, 37R;
(5) 25L, 26T, 37R;
(6) 12V, 26L, 37R;
(7) 12R, 37V;
(8) 12T, 25T, 26Q, 37K;
(9) 12T, 25C, 37N;
(10) 12T, 25A, 26M, 37N;
(11) 12D, 25C, 37N;
(12) 12A, 25L, 26M, 37R;
(13) 12L, 25Y, 27F, 37F;
(14) 12L, 25A, 26D, 31D:
(15) 12P, 25P, 26V, 31A;
(16) 12L, 25S, 26Q, 33E;
(17) 12A, 19C, 25C, 26M, 37N;
(18) 12T, 25A, 31H, 37N;
(19) 12T, 25A, 37N, 45L; or
(20) 12T, 25C, 29L, 35L, 37N.

In various embodiments, the above sets of substitutions (1)-(6), (8)-(12) and (17)-(20) are relative to SEQ ID NO:5 (hMeCP2); set of substitution (13) is relative to SEQ ID NO:3 (hMBD3); and sets of substitutions (7) and (14)-(16) are relative to SEQ ID NO:2 (hMBD2). Generally, all substitutions in position 26 disclosed herein are not preferred in the reference sequence set forth in SEQ ID NO:3, while all substitutions in position 27 disclosed herein are preferred in said reference sequence of SEQ ID NO:3 but not in all other human sequences set forth in SEQ ID Nos. 1-2 and 4-5.

In various embodiments, to ensure MBD functionality, some of the original amino acid positions are not altered but maintained. In the following, some of these consensus sequences are disclosed.

In various embodiments, the MBD core domain of the isolated MBD variant comprises any one or more, preferably at least 2, more preferably at least 3, most preferably all 4, of the amino acids 14R/K, 24D/E, 36R/K and 40E/Q/D, optionally any one or more, preferably at least 2, more preferably at least 3, most preferably all 4, of the amino acids 14R/K, 24D/E, 27Y/F/L, 36R/K and 40E/Q/D/S, using the positional numbering of SEQ ID NO:1. In some embodiments, one or more, preferably at least 2, more preferably at least 3, most preferably all 4, of the amino acids 14R/K, 24D/E, 36R/K and 40E/Q/D/S, optionally any one or more, preferably at least 2, more preferably at least 3, most preferably all 4, of the amino acids 14R/K, 24D/E, 27Y/F/L, 36R/K and 40E/Q/D/S, are present in addition to the at least one amino acid substitution selected from 12S, 12T, 25I, 25T, 25A, 26T, 37N and 37K using the positional numbering of SEQ ID NO:1. These amino acids at positions 14, 24, 27, 36 and 40 using the positional numbering according to SEQ ID NO:1 correspond to the amino acids at the corresponding positions in the wildtype MBD core domain and are thus in some embodiments unaltered, i.e. not substituted. This can help to ensure MBD functionality.

In various embodiments, the isolated MBD variant comprises any one or more, preferably at least 4, more preferably at least 6, even more preferably at least 8, most preferably at least 10, of the amino acids 1P/K, 3L/V, 6G/D, 7W/F, 8R/Q/K/E/T, 9R/K, 17G, 27Y/F/L, 30P, 32G, 44Y/F and 45L/I/F, preferably any one or more, preferably at least 5, more preferably at least 10, even more preferably at least 15, most preferably at least 20, of the amino acids 1P/K, 2A/S/T, 3L,V, 4G/P, 5P/Q/C/E, 6G/D, 7W/F, 8R/Q/K/E/T, 9R/K, 10R/E/V/K, 11E/V/L, 12V/K, 13F/I/P/Q, 14R/K, 15K/R/L, 16F/S, 17G, 18A/L/K/R, 19T/S, 20C/A, 21G, 22R/K/H, 23S/R/F/Y, 24D/E, 25T/V, 26Y/F, 27Y/F/L, 28Q/F/Y/I, 29S/N, 30P, 31T/S/Q, 32G, 33D/K/L, 34R/K/A, 35I/F, 36R/K, 37S, 38K, 39V/P/S, 40E/Q/D/S, 41L, 42T/A/I, 43R/N/A, 44Y/F/V, 45L/I/F, preferably using the positional numbering of SEQ ID NO:1. This is the full consensus sequence of the MBDs disclosed herein. It is understood that the feature that the MBD variants comprise an amino acid substitution at at least one of the positions corresponding to positions 12, 14, 19, 21, 22, 23, 24, 25, 26, 27, 29, 31, 33, 35, 36, 37, 38, 39, 40 and 45, for example
12, 14, 19, 21, 22, 23, 24, 25, 26, 27, 35, 36, 37, 38, 39 and 40; or
12, 19, 21, 25, 26, 27, 29, 31, 33, 35, 36, 37, 38, 39, 40 and 45; or
12, 19, 21, 22, 23, 25, 26, 27, 35, 36, 37, 38, and 39; or
12, 19, 21, 25, 26, 27, 29, 31, 33, 35, 36, 37, 38 and 45; or
12, 25, 26, 27, 29, 31, 33, 35, 37 and 45; or
12, 25, 26, 27, 36 and 37; or
12, 25, 26, 37, according to SEQ ID NO:1 means that the above amino acid in the consensus sequence is replaced by another amino acid, which is typically not a consensus amino acid. This may mean, for example and in some embodiments, that if the amino acid substitution is in position 29, the target amino acid is preferably not S (including if the starting amino acid is N) or N (including if the starting amino acid is S). In various embodiments, the target amino acid of any of the substitutions according to the invention is thus not any one listed above as a consensus amino acid. This may, for example, also apply to 12V.

In various embodiments, the MBD core domain of the isolated MBD variant comprises at least one amino acid substitution selected from 12V, 12S, 12T, 12A, 12R, 12D, 12L, 12P, 25I, 25T, 25A, 25C, 25L, 25Y, 25P, 25S, 26T, 26S, 26F, 26L, 26D, 26V, 26Q, 26M, 27F, 29L, 31A, 31D, 31H, 33E, 35L, 36C, 37N, 37K, 37Q, 37R 37V, 37F, and 45L, preferably 12V, 12S, 12T, 12A, 12R, 12D, 12L, 12P, 25I, 25T, 25A, 25C, 25L, 25Y, 25P, 25S, 26T, 26S, 26F, 26L, 26D, 26V, 26Q, 26M, 27F, 29L, 31A, 31D, 31H, 33E, 37N, 37K, 37Q, 37R 37V, 37F, and 45L, more preferably 12V, 12T, 12A, 12R, 12D, 12L, 12P, 25T, 25A, 25C, 25L, 25Y, 25P, 26T, 26S, 26F, 26L, 26D, 26V, 26Q, 26M, 27F, 29L, 31A, 31D, 31H, 33E, 35L, 36C, 37N, 37K, 37Q, 37R 37V, 37F, and 45L using the positional numbering of SEQ ID NO:1, while retaining consensus amino acids according to the above list in all other positions. Typically, these MBD variants comprise an MBD core domain that has preferably at least 65, 70, 75 or 80 %, sequence homology or identity relative to any one of SEQ ID Nos.1-45, preferably to any one of SEQ ID Nos. 1-28, more preferably to SEQ ID Nos. 1-10, most preferably to SEQ ID NO: 1, 2, 3, 4, or 5.

Again, the above listed amino acids for positions 1-45 correspond to the amino acids at the corresponding positions in the wildtype MBD core domain, i.e. form a consensus sequence of the wildtype MBD core domain, and are thus in some embodiments unaltered, i.e. not substituted. It is understood that when some of these positions overlap with those that are disclosed herein as optionally being substituted, such as positions 12, 14, 19, 25, etc., that the substitutions prevail. In all embodiments, it is, however, preferred that the non-substituted positions are occupied by amino acids as listed above. This means, for example, that if position 25 is not substituted, it is preferably T or V, which typically corresponds to the respective wildtype sequence.

In various embodiments, the polypeptide comprising the MBD domain may comprise at least one amino acid substitution in the amino acid sequence N- or C-terminal to the MBD core domain, preferably C-terminal to the MBD core domain, in particular in position 158 of hMeCp2[90-181] (SEQ ID NO:69) (16 amino acids C-terminal to amino acid 45 of the MBD core domain of SEQ ID NO:5 (position 61). Most preferably, the amino acid sequence of variant T158M comprises or consists of the amino acid sequence set forth in SEQ ID NO:76. In such embodiments, the MBD core domain of the isolated MBD variant may or may not comprise a substitution, as described above. It is generally understood that all MBD domain variants disclosed herein may additionally comprise flanking amino acid sequences, i.e. amino acid sequences that are located directly N- and/or C-terminal to the MBD core domain as defined herein. These sequences may correspond to the respective wildtype template sequences or may be different, heterologous or mutated sequences. In various embodiments, such polypeptides comprising the MBD core domain as defined herein are at least 50 amino acids in length, for example 60, 70, 80, 90, 100 or more amino acids. In addition to the MBD domain, these polypeptides may comprise further domains, such as those naturally occurring in the corresponding wildtype polypeptides.

The present invention relates to isolated MBD variants that have relative to the corresponding wildtype MBD an altered affinity for a DNA molecule comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC). In various embodiments, these variants are those described above having amino acid substitutions at the indicated positions.

Generally, in various embodiments, all isolated MBD variants disclosed herein may have an altered binding affinity, preferably increased affinity, for CpG dinucleotides and their complement in which
(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);
(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC);
(l) both cytosine bases are 5-carboxylated (caC);
(m) one cytosine base is 5-methylated cytosine (mC) and the other is non-modified (C); and/or
(n) both cytosine bases are 5-methylated (mC/mC).

In various other embodiments, the isolated MBD variant has differential binding affinity for any two CpG dinucleotides and their complement selected from the following oxidized 5-methylated cytosine configurations:
(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);
(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC); and/or
(l) both cytosine bases are 5-carboxylated (caC).

According to the present invention, the DNA molecule may be single-stranded or double-stranded, wherein double-stranded DNA is preferred. In a preferred embodiment, the DNA molecule is in its native state and is not chemically modified by any pretreatment.

Preferably, the cytosine nucleobases selected from the group consisting of cytosine (C), 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), in the CpG dinucleotide of interest and its complement are physiological cytosine states known in the art.

In various embodiments, the DNA molecule(s) can be labelled, preferably fluorescently labelled. In various embodiments, the labelled DNA molecules may have different oxi-mC configurations in a single CpG.

In various preferred embodiments, the isolated MBD variant comprises or consists of the amino acid sequence set forth in SEQ ID Nos. 46 to 60 and/or 61-67.

The invention further encompasses a conjugate comprising the isolated MBD variant as described above.

The term "conjugate" refers to a molecule that comprises the isolated MBD variant as described above coupled, typically covalently coupled, to another moiety, such as another protein, peptide, polypeptide, affinity ligand or detectable label or other moiety.

Such "moieties" are not particularly limited and can be easily chosen by a person skilled in the art in accordance with the particular application of interest. Further examples of such moieties include fluorescent dyes, fluorescent proteins such as e.g. green fluorescent protein (GFP), affinity ligands such as e.g. biotin, digoxigenin, dinitrophenol, magnetic particles, antibodies, including antibody fragments and antibody mimetics known in the art, protein tags, and proteins having enzymatic activities such as nuclease activities. Suitable protein tags in this respect include e.g. 18A-Tag, ACP-Tag, Avi-Tag, BCCP-Tag, Calmodulin-Tag (CaM-Tag), Chitin-binding-Protein-Tag (CBP-Tag), E-Tag, ELK16-Tag, ELP-Tag, FLAG-Tag, Flash-Tag, poly-glutamic acid-Tag, Glutathion-S-Transferase-Tag (GST-Tag), Green fluorescent protein-Tag (GFP-Tag), Hemagglutinin-Tag (HA-Tag), poly-Histidin-Tag (His-Tag), Isopeptag, Maltose binding protein-Tag (MBP-Tag), Myc-Tag, Nus-Tag, ProtA-Tag, ProtC-Tag, S-Tag, SBP-Tag, Snap-Tag, SpyTag, SofTag 1 and 3, Streptavidin-Tag (Strep-Tag), Strep-II-Tag, Tandem Affinity Purification-Tag (TAP-Tag), TC-Tag, Thioredoxin-Tag (TRX-Tag), Ty-Tag, V5-Tag, VSV-Tag, and Xpress-Tag, which are known in the art. Such "coupling moieties" can be coupled directly or indirectly to the MBD variant, or can be expressed as part of a conjugate comprising also the MBD variant. Methods for the coupling of respective "coupling moieties" and for the expression of respective conjugates are not particularly limited and are known in the art. For example, they include methods employing a His-Tag on the expressed conjugates and purification via NiNTA as known in the art, without being limited to this method.

In this context, MBD variants or conjugates can include non-natural amino acids with reactive groups. The coupling of the above compounds can be effected e.g. via click chemistry at said reactive groups. Suitable reactive groups in this context are not particularly limited and include e.g. azides, alkynes, alkenes, including strained alkynes and alkenes that have been genetically encoded in the form of non-natural amino acids.

Applications of respectively coupled MBD variants are not particularly limited and can be easily devised by the person skilled in the art in accordance with the respective application or scientific problem to be addressed.

In various embodiments, the conjugate further comprises an enzyme, preferably a nucleic acid modifying enzyme such as a nuclease (Fokl nuclease, Micrococcal nuclease, DNasel or other endo- and exonucleases), a methyltransferase (DNMT1, DNMT3a, DNMT3b, M.Sssl, Daml or other Mtases known in the art), a deaminase, a biotin-ligase (BirA) or others. In another embodiment, the conjugate comprises a tag for later detection (e.g. SNAP tag, Sun tag, Flag tag, HA tag, His tag, etc.), or a fluorophore (e.g. GFP, mCherry, mClover, BFP, mRuby, DsRed etc.), which is bound to the isolated MBD variant.

Preferably, the isolated MBD variant or the conjugate as described above can be used for the determination of the methylation state of cytosine residues and/or oxidation state of 5-methylated cytosine residues in a CpG dinucleotide of interest and its complement in a DNA molecule or for the enrichment of DNA molecules comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

Accordingly, the invention also relates to the use of the isolated MBD variant as described above or the conjugate as described above for the determination of the methylation state of cytosine residues and/or oxidation state of 5-methylated cytosine residues in a CpG dinucleotide of interest and its complement in a DNA molecule or for the enrichment of DNA molecules comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

Further encompassed is a method for the determination of the methylation state of cytosine residues and/or oxidation state of the methyl group of 5-methylated cytosine residues in a CpG dinucleotide of interest and its complement in a DNA molecule, said method comprising
(a) providing a molecular probe comprising a Methyl-CpG binding domain (MBD) that binds to the region of the DNA molecule comprising said CpG dinucleotide of interest and its complement and differentially binds to different methylated cytosine and/or oxidized 5-methyl-cytosine configurations in the CpG dinucleotide of interest and its complement, wherein said differential binding is detectable by differences in binding affinity;
(b) determining the methylation state of said cytosine residues and/or oxidation state of said 5-methylated cytosine residues in said CpG dinucleotide of interest by contacting the molecular probe with the DNA molecule and determining the binding affinity of the molecular probe to the region of the DNA molecule comprising said CpG dinucleotide of interest.

Preferably, in the method described above, the determination of the methylation state of cytosine residues and/or oxidation state of the methyl group of 5-methylated cytosine residues in a CpG dinucleotide of interest and its complement in a DNA molecule comprises determining the presence or the level of a nucleobase selected from the group consisting of cytosine (C), 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), in the CpG dinucleotide of interest and its complement.

The binding affinity can be determined from a recombinantly expressed, isolated, and purified protein containing said MBD core domain using for example an electrophoretic mobility shift assay (EMSA) with fluorescently or radioactively labelled probes that contain a single CpG dinucleotide with one of the modification states specified above. Typically, the probe is added in nanomolar concentrations, e.g. 2 nM, and the MBD protein in micromolar to nanomolar concentrations, e.g. 1,024 nM or 128 nM (e.g. FIG. 6D) [7,8].

The selectivity of an immobilized MBD can also be determined using a mixture of said DNA probes of which at least one DNA probe is labelled with a different/distinguishable label such as a different fluorophore or other. Immobilization can be performed on cell surfaces, e.g. the bacterial cell surface, or on beads for purified proteins. In combination with fluorophore-labelled probes, a microfluidic or a flow cytometer enabled for fluorescence-activated cell sorting (FACS) can be used to quantify the selectivity of the MBD domain. If the detectable label is a unique barcode, e.g. a unique DNA sequence, the determination of binding selectivity can take place using a next-generation sequencing platform.

In various embodiments of the methods disclosed herein, the MBD variant and the DNA molecules are added in a molar ratio of MBD to DNA of between 2:1 and 500:1, more preferably of between 30:1 to 70:1, more preferably of between 40:1 to 60:1, most preferably of 50:1.

The invention further relates to a method for the enrichment of DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), said method comprising
(a) providing a molecular probe comprising a Methyl-CpG binding domain (MBD) variant according to the invention that binds to the region of the DNA molecule comprising said CpG dinucleotide of interest and differentially binds to different methylated cytosine and/or oxidized 5-methyl-cytosine configurations in the CpG dinucleotide of interest and its complement, wherein said differential binding is facilitated by differences in binding affinity;
(b) contacting the molecular probe with a sample comprising DNA molecules comprising said CpG dinucleotide of interest and its complement under conditions that allow binding of the molecular probe to its target;
(c) enriching the DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), by separating the DNA molecules based on their affinity for the molecular probe.

In preferred embodiments, the binding affinity of the variant MBD to the DNA molecule comprising specific cytosine nucleobases as described above in a CpG dinucleotide differs from the binding affinity of the wildtype MBD to the DNA molecule comprising the same specific cytosine nucleobases in the CpG dinucleotide by at least 1%, preferably at least 5 %, more preferably at least 10 %, even more preferably at least 20 %, or even more preferably 50 % or more. The affinity may be expressed in terms of the dissociation constant Kd, as can for example be determined by various methods known in the art, including the aforementioned electromobility shift assays, fluorescence polarization assays, ELISA, footprinting, surface plasmon resonance based assays, microscale thermophoresis, calorimetric and other spectroscopic methods.

Preferably, the molecular probe of the method for the enrichment is immobilized on a substrate, more preferably on a functionalized capture bead. The molecular probe may thus comprise an affinity ligand that allows immobilization of the molecular probe on a substrate.

Affinity ligands for use according to these embodiments are not particularly limited and are known in the art. They include for example biotin, digoxigenin, dinitrophenol, magnetic particles, antibodies, including antibody fragments and antibody mimetics, and protein tags, e.g. as indicated above. Methods for the direct or indirect coupling of affinity ligands to MBD variants are not particularly limited and are known in the art. In this context, the term "coupled directly or indirectly" expressly includes the expression of conjugate molecules of MBD proteins and any particular proteinaceous affinity ligands. Further, the term "coupled indirectly" includes any coupling strategies employing linkers, binding via further affinity partners, and the like, which are known in the art.

In various embodiments, the enrichment step of the enrichment method comprises separating the complexes of the DNA molecules with the immobilized molecular probe from the non-complexed DNA molecules, the enrichment optionally including chromatography, centrifugation or magnetic bead separation.

In various other embodiments, the enrichment step may also comprise methods of DNA depletion. Particular methods include the use of His-tags as affinity ligand and depletion of DNAs bound to respective variant MBDs via Ni-NTA-beads, as well as the use of other protein tags as indicated above in connection with the respective affinity binding partners.

For example, DNA molecules in which specific (modified) cytosine nucleobases or specific oxidation states of the methyl group of 5-methylated cytosine residues in a CpG dinucleotide of interest and its complement are absent (or present) are depleted from a mixture containing DNA molecules in which the cytosine nucleobases are present, as well as DNA molecules in which the cytosine nucleobases are absent. In this manner, DNA molecules in which the cytosine nucleobases are present (or absent) are enriched in the mixture. Therefore, the MBD variant may be coupled directly or indirectly to a protein having nuclease activity, said protein allowing for the differential digestion of DNA molecules in which the cytosine nucleobases of interest are absent, wherein DNA molecules in which epigenetic modifications of said nucleobases of interest are present remain undigested.

Proteins having nuclease activity for use according to this embodiment are not particularly limited and are known in the art. They include for example restriction enzymes and cleavage domains of restriction enzymes, e.g. the cleavage domain of Fokl. Methods for the direct or indirect coupling of proteins having nuclease activity to MBD variants are not particularly limited and are known in the art. In this context, the term "coupled directly or indirectly" expressly includes the expression of conjugate molecules of MBD variants and any particular proteins having nuclease activity. Further, the term "coupled indirectly" includes any coupling strategies employing linkers, binding via further affinity partners, and the like, which are known in the art. Moreover, methods for the digestion of DNA molecules in which specific modification (or a specific oxidation state) of said nucleobases is absent using said proteins having nuclease activity are not particularly limited and are known in the art. In this manner, DNA molecules in which the cytosine nucleobases of interest are present are enriched in the mixture.

The enriched DNA molecules, in which the cytosine nucleobases of interest are present, obtained by the method as described above, can be further used for any particular application of interest. For examples, said DNA molecules can be used for sequencing applications known in the art, including PCR and so-called Next Generation Sequencing methods, microarray analyses, or any methods for the analysis of nucleic acids known in the art.

The methods of the present invention can be used for the concurrent determination of the presence or absence of more than one particular cytosine nucleobase of interest in a given DNA molecule. In particular, several aliquots of a given DNA molecule can be provided, and the presence of different cytosine nucleobases of interest can be determined, respectively. As an example, the method of the present invention can be performed with respect to the detection of C, mC, hmC, fC, and caC, respectively, for a given DNA molecule.

Preferably, the difference in binding affinity of the molecular probe is an increased binding affinity for a methylated state of cytosine and/or an oxidized state of 5-methylated cytosine, in particular 5-hydroxymethylcytosine, relative to the non-methylated cytosine and/or the non-oxidized 5-methylated cytosine.

Unless otherwise defined, all technical and scientific terms used herein have the same meaning as commonly understood by one of ordinary skill in the art. The singular terms "a", "an" and "the" include plural referents unless context clearly indicates otherwise. Similarly, the word "or" is intended to include "and" unless the context clearly indicates otherwise. The term "comprises" means "contains" or "includes". In case of conflict, the present specification, including explanations of terms, will control.

It is to be understood that the individual embodiments described herein can be combined with other embodiments of this application to form new embodiments, even if they were not disclosed contiguously. All embodiments and examples described for the MBD variant according to the invention also apply to the conjugate, use and/or method according to the invention, and vice versa. Additionally, it is to be understood that the various or preferred embodiments serve as examples and do not limit the scope of the invention.

The present invention will be further illustrated in the following non-limiting examples.

### EXAMPLES

### Example 1

### Cloning of Expression and Display Vectors

The MBD expression plasmids were derived from pET-21d(+) (Merck, Darmstadt, Germany), digested with *Xho*I and Ncol (New England Biolabs) to replace the T7 tag by Gibson assembly with a FLAG peptide-maltose-binding protein (MBP) tag, a factor Xa and a TEV recognition and cleavage site. The resulting vector pBeB1379 allowed expression of N-terminal MBP-MBD fusion proteins with a non-cleavable C-terminal 6xHis tag for purification.

The MBD surface display plasmid pBeB1383 was derived from a pBeB1379 digest with *Xho*I and Ndel to replace the FLAG-MBP tag, the factor Xa and the TEV recognition and cleavage site with an AIDA-I surface display cassette by Gibson assembly.

Both pBeB1379 and pBeB1383 contained a suitable multiple cloning site to incorporate, exchange, or isolate the coding sequences of said MBD variants.

### Expression of MBD proteins

Similar to a protocol of [9], expression plasmids were transformed into E. *coli* BL21-Gold(DE3) (Agilent), and fresh overnight cultures of single clones were diluted to an optical density (OD₆₀₀) of 0.05 in 30 mL LB-Miller broth supplemented with 50 µg/mL carbenicillin, 1 mM MgCl₂ and 1 mM ZnSO₄. Cultures were grown at 37 °C (220 rpm) to an OD₆₀₀ of 0.5-0.6, briefly chilled on ice, and then induced by supplying 1 mM isopropyl β-d-1-thiogalactopyranoside (IPTG). Cultures were incubated at 25 °C (150 rpm) for at least 6 h or overnight, and cells were harvested and washed once by resuspension in 0.25 vol ice-cold 20 mM Tris-HCl (pH = 8.0). Pellets were resuspended in 2 mL binding buffer (20 mM Tris-HCl, 250 mM NaCl, 10% glycerol, adjusted to pH = 8.0, supplemented with 10 mM 2-mercaptoethanol, 5 mM imidazole and 0.1 % Triton X-100), and sonicated in a Bioruptor Pico (Diagenode) at 4 °C using 3 x 4 cycles (30 s pulse of 20-60 kHz, 25-200 W and 30 s rest). Suspensions were treated with 0.1 mg/mL lysozyme (Merck) and 10 U/mL DNase I (New England Biolabs) overnight. After centrifugation at 14,000 x g for 20 min at 4 °C, the cleared supernatants were retained, diluted with 1 vol binding buffer, mixed with 450 µL 50 % Ni-nitriloacetic acid (NTA) agarose resin (ThermoFisher), and incubated at 4 °C for 2 h. The resins were washed 2 x with 1 mL binding buffer containing 90 mM imidazole (20 min at 4 °C) and the fusion proteins were eluted in 2 x 0.2 mL and 1 x 0.4 mL binding buffer with 500 mM imidazole (10 min at 4 °C). Pure fractions (SDS PAGE) were combined and dialyzed against 3 x 15 mL 20 mM HEPES, 100 mM NaCl, 10% glycerol, adjusted to pH = 7.3, and 0.1% Triton X-100 in Slide-A-Lyzer MINI devices (3.5 kDa MWCO, ThermoFisher). An additional 1:2-1:5 dilution is recommended when scaling up this procedure to avoid precipitation during dialysis. The protein concentrations were determined with a BCA assay (ThermoFisher) and the proteins stocked at 15 µM after snap freezing in liquid nitrogen, at -80 °C (stable for several months).

### Electrophoretic Mobility Shift Assays

24-mer oligodeoxynucleotide (ODNs) pairs containing one of the specific combinations of modified cytosine residues were combined at 1.5 µM of the labeled strand and 1.8 µM of the unlabeled strand in rudimentary EMSA buffer (20 mM HEPES, 30 mM KCI, 1 mM EDTA, 10 mM (NH₄)₂SO₄, pH = 7.3), incubated at 95 °C for 5 min, slowly brought to room temperature in a water bath for duplex formation, and subsequently diluted to 30 nM with respect to the labeled strand. The non-specific binding trap duplex was prepared by annealing a 24-mer poly(dA) with a 24-mer poly(dT) at equimolar ratios of 50 µM. EMSA were carried out according to a well-established protocol [9]. In brief, purified MBDs were diluted to 0, 2, 4, 8, 16, 32, 64, 128, 256, 512, and 1,024 nM in dialysis buffer with 0.1 mg/mL BSA (New England Biolabs) and incubated with 2 nM labeled duplex and 50 ng/µL poly(dA)·poly(dT) in EMSA buffer containing 1 mM dithiothreitol and 0.2 % Tween 20 in a final volume of 15 µL. The binding was allowed to equilibrate for 20 min at room temperature before 3 µL of a 6x loading dye (1.5 x TBE, pH = 7.5, 40% glycerol, 70 pg/mL bromophenol blue) were added on ice. These samples (10 µL) were loaded on pre-run 0.25 x TBE, 12% polyacrylamide gels and run at 240 V for 45 min at 4 °C in Mini-PROTEAN vertical electrophoresis cells (Bio-Rad). Gels were recorded on a Typhoon FLA-9500 laser scanner (GE Healthcare) equipped with a 473 nm laser and a 510 LP filter at 700-800 V PMT amplification without over-exposure. The fraction of bound duplex was determined using ImageQuant TL v8.1 1D Gel Analysis (GE Healthcare) applying rubber band background subtraction and manual peak detection with approximately equal peak areas across all lanes. The fraction of bound probe serves as a means to quantitate the relative affinity of a MBD at a fixed protein concentration (FIG. 6A). The removal of the N-terminal solubility tag such as MBP is optional and does not affect binding selectivity at the concentrations typically used in such assays.

### Surface Display Assay

24-mer oligodeoxynucleotide (ODNs) pairs containing one of the specific combinations of modified cytosine residues and a 5'-biotin label were combined at equimolar ratios, annealed as described, and diluted to a final concentration of 400 nM in 6 x EMSA buffer and 300 ng/µL poly(dA)·poly(dT). Individual probes were labelled with fluorescent streptavidin (SAv) conjugates such as SAv-(R)-phycoerythrin (BioLegend) or SAv-AF488 (ThermoFisher) at 1.5-fold excess over the biotin component. Excess streptavidin was quenched after labeling at room temperature by addition of a 20-fold excess of free biotin (Car Roth). Surface display plasmids were transformed into E. *coli* BL21-Tuner(DE3) strains (Novagen) and fresh overnight cultures of single clones were diluted to an optical density (OD₆₀₀) of 0.05 in 3 mL LB-Miller broth supplemented with 50 µg/mL carbenicillin. Surface display was induced with 50 µM isopropyl β-d-1-thiogalactopyranoside (IPTG) at an OD600 of 0.5-0.7 for60 min at 30 °C. An amount of cells corresponding to an OD600 of 0.04 was harvested, washed and resuspended in 20 µL phosphate buffered saline (PBS) to which 10 µL of the staining mix was added which was prepared as described above according to the experimental demands. For example, this staining mix could contain 14 different ODNs labelled with SAv-AF488 and one ODN labeled with SAv-(R)-phycoerythrin at equimolar ratio. After 20 min at 22 °C, 700 rpm, the cells were washed again with PBS and analyzed on a flow cytometer (Sony Biotechnology) equipped with a 488 nm and 562 nm laser and suitable filter optics.

### Binding Assay Validation

Both assays, the electrophoretic mobility shift assay (EMSA) and the surface display assay on FACS result in comparable estimates of the binding selectivity for a known binding MBD such as hMBD2 [2-81] containing SEQ ID NO:2 (FIG. 6B) as well as a known non-binding MBD such as hMBD3 [2-81] containing SEQ ID NO:3 (FIG. 6C) with the dynamic range of the EMSA at low protein concentrations, e.g. 128 nM (FIG. 6D) being most similar to the behavior observed under the conditions of said surface display assay (FIG. 6E).

### Identification of MBDs with Novel Binding Selectivity

Immobilization on the bacterial cell surface was used to purify candidate binders from codon-degenerated MBD proteins containing SEQ ID Nos. 1, 2, 3, 4, or 5 using a modified procedure of the surface display assay described above. Codons corresponding to positions 12, 25, 26 (not SEQ ID NO:3), 27 (SEQ ID NO:3 only), and 37 were randomized using NNK-degenerated primers and standard molecular biology and protein engineering technologies. Individual clones harboring a single MBD variant of said libraries were sub-cloned in an expression plasmid such as pBeB1379, the protein product expressed and purified and subjected to EMSA (FIG. 7 and FIG. 8).

### Results

- F105 is a MBD variant of SEQ ID NO:5 with selectivity for mC/caC, mC/mC, fC/caC and caC/caC.
- F106 is a MBD variant of SEQ ID NO:5 with selectivity for mC/hmC and mC/mC. It is the first example, in which an engineered MBD has higher affinity towards a CpG containing an oxidized mC species than for mC/mC (FIG. 9).
- F271 is a MBD variant of SEQ ID NO:5 with a single, high selectivity for mC/mC. This is a novel property within the domain of human MBDs containing SEQ ID Nos. 1, 2, 3, 4, or 5.
- F272 is a MBD variant with selectivity for a CpG containing one or two caC modifications in a single CpG. The most pronounced binding affinity is towards mC/caC.
- F273 is a MBD variant of SEQ ID NO:5 with selectivity for a CpG containing one or two caC modifications in a single CpG. The most pronounced binding affinity is towards caC/caC.
- F274 is a MBD variant of SEQ ID NO:5 with selectivity for mC/mC and mC/hmC within the domain of hemi-modified mC-containing CpGs as well as forhmC/hmC.
- F275 is a MBD variant of SEQ ID NO:2 with selectivity for mC/mC or a CpG containing one or two fC modifications in a single CpG.

Furthermore, within the set of codon-degenerated positions 12, 25, 26, 27, and 37, and MBD variants based on SEQ ID Nos. 2, 3, and 5 unpurified further variants with potentially high selectivity for mC/hmC, mC/fC, caC/caC and mC/caC were identified (FIG. 10).

The above results show that rationally identified positions within the MBD have the potential to alter the selectivity of various naturally occurring MBD protein domains for oxidized mC species in single CpGs. This enables both, the engineering of MBDs that are highly selective as compared to wildtype MBDs (e.g. F271) as well as MBDs that have a novel specificity with regards to the oxidation state of the cytosine bases in the CpG, i.e. MBDs with strongly reduced affinity for mC/mC, but increased affinity towards other configurations such as mC/hmC (e.g. F106), mC/caC (e.g. F272 or F273).

The amino acid sequences of the MBD core domain of variants F105, F106, F271, F272, F273, F274 and F275 together with further optimized variants thereof are shown in FIG. 5.

### Example 2

### Data analysis and Kd determinations

All data was curated and analyzed with R v3.6.0. Given a single ligand binding site of the MBD protein domain and the absence of additional intramolecular interactions, the relationship between the total concentration of labeled duplex [L]₀, added to the reaction, the total concentration of MBD [R]₀, the fraction of bound ligand [RL]/[L]₀ at equilibrium and the dissociation constant Kd follows the quadratic equation [10] ${\left[RL\right]}^{2} - \left[RL\right] \left({\left[R\right]}_{0}+{\left[L\right]}_{0}+{K}_{d}\right) + {\left[R\right]}_{0} {\left[L\right]}_{0} = 0$ which has been used to determine the K_{d} of MBD protein domains from fractional binding by Khrapunov *et al.* [11] as well as from free ligand, [L] = [L]₀ - [RL], by Yang *et al.* [12]. Here, we use the solution for the experimentally observed fraction of bound ligand, [RL]/[L]₀, which is $\left[RL\right] / {\left[L\right]}_{0} = \left({\left[R\right]}_{0}+{\left[L\right]}_{0}+{K}_{d}-{\left[{\left({\left[R\right]}_{0}+{\left[L\right]}_{0}+{K}_{d}\right)}^{2}-4{\left[R\right]}_{0}{\left[L\right]}_{0}\right]}^{1 / 2}\right) / \left(2{\left[L\right]}_{0}\right)$ to determine the K_{d} by non-linear curve fitting using the Levenberg-Marquardt algorithm. We propagated the uncertainty associated with the K_{d} estimates to the derived fold-changes according to the basic equation for error propagation [13].

### Results

MBDs showed markedly different selectivity profiles for differentially modified CpGs despite their high degree of sequence conservation, particularly at residues interacting with the CpG. Therefore, it is of interest how RTT-associated single amino acid substitutions in MeCp2 would affect the selectivity profile of its MBD.

The mutants L124F, T158M, R133C and S134C [14] of human MeCp2[90-181] (wildtype sequence is set forth in SEQ ID NO:69 (FIG. 16)) were evaluated using EMSA and affinity measurements (Table 1 and FIG. 11). The single amino acid substitutions L124F (position 27 of SEQ ID NO:5), R133C (position 36 of SEQ ID NO:5) and S134C (position 37 of SEQ ID NO:5) are within the MBD core domain of hMeCp2 as defined herein (FIG. 12A). The single amino acid substitution T158M is present C-terminal to the MBD core domain as defined herein (position 61) (FIG. 12B). The MBD core sequence of said mutants is set forth in SEQ ID Nos. 73-76.

**Table 1: Dissociation constants K_{d}/nM**

| **duplex** | **wildtype** | **R133C** | **S134C** |
|---|---|---|---|
| mC/mC | 27±4 | 118±12 | 117±9 |
| mC/hmC | 224±25 | 660±70 | 1,150±70 |
| mC/fC | 197±24 | 540±50 | 790±50 |
| hmC/hmC | 970±90 | 2,560±260 | 7,600±600 |
| hmC/fC | 540±60 | 1,630±90 | 4,600±400 |
| C/hmC | 1,080±100 | 8,100±500 | 7,600±700 |

### REFERENCES

[1] M.J. Booth, M.R. Branco et al., Science 336(6083), 934-937 (2012).
[2] M. Yu, G.C. Hon et al., Cell 149(6), 1368-1380 (2012).
[3] M.J. Booth, G. Marsico et al., Nat Chem 6(5), 435-440 (2014).
[4] F. Neri, D. Incarnato et al., Cell Rep 10(5), 674-683 (2015).
[5] L. Shen, H. Wu et al., Cell 153(3), 692-706 (2013).
[6] H. Wu and Y. Zhang, Genes Dev. 25(23), 2436-2452 (2011).
[7] H. Hashimoto, J.E. Pais et al., Nature 506(7488), 391-395 (2014).
[8] V. Valinluck, H. Tsai et al., Nucl Acids Res 32(14), 4100-4108 (2004).
[9] A. Free et al., J Biol Chem 276, 3353-3360 (2001).
[10] E. C. Hulme, M.A. Trevethick, Br J Pharmacol 161, 1219-1237 (2010).
[11] S. Khrapunov et al., Biochemistry 53, 3379-3391 (2014).
[12] Y. Yang, T. G. Kucukkal et al., ACS Chem Biol 11, 2706-2715 (2016).
[13] P.R. Bevington, McGraw-Hill Education Ltd, (1969).
[14] J. Christodoulou, A. Grimm et al., Human mut 21, 466-472 (2003).

## Claims

1. Isolated Methyl-CpG binding domain (MBD) variant, comprising an MBD core domain that has at least 85 % sequence identity relative to any one of SEQ ID Nos.1-28 and comprising at least one amino acid substitution relative to the corresponding wildtype MBD as set forth in any one of SEQ ID Nos. 1-28 in at least one of the positions corresponding to positions 12, 25, 26 and 37, in SEQ ID NO:1, wherein said at least one, preferably at least two or at least three, amino acid substitution(s) in at least one, preferably in at least two or at least three, of the positions corresponding to positions 12, 25, 26 and 37 is selected from 12V, 12T, 12A, 12R, 12D, 12L, 12P, 25T, 25A, 25C, 25L, 25Y, 25P, 25S, 26T, 26S, 26F, 26L, 26D, 26V, 26Q, 26M, 37N, 37K, 37Q, 37R, 37V and 37F, preferably 12V, 12T, 12A, 12R, 25T, 25A, 25C, 25L, 26T, 26S, 26F, 26L, 26Q, 37N, 37K, 37Q, 37R and 37V, most preferably 12V, 12T, 25T, 25A, 25C, 26T, 26S, 26L, 26Q, 37N, 37R, and 37K using the positional numbering of SEQ ID NO:1, wherein said MBD variant has relative to the corresponding wildtype MBD an altered affinity for a DNA molecule comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

2. The isolated MBD variant of claim 1, wherein said variant comprises any two or more, preferably three or all, of the substitutions set forth in the following sets of substitutions:
(1) 12T, 25T, 26T, 37K;
(2) 12T, 25A, 37N;
(3) 25C, 26S, 37Q;
(4) 12A, 25C, 26F, 37R;
(5) 25L, 26T, 37R;
(6) 12V, 26L, 37R;
(7) 12R, 37V;
(8) 12T, 25T, 26Q, 37K;
(9) 12T, 25C, 37N;
(10) 12T, 25A, 26M, 37N;
(11) 12D, 25C, 37N;
(12) 12A, 25L, 26M, 37R;
(13) 12L, 25Y, 27F, 37F;
(14) 12L, 25A, 26D, 31D:
(15) 12P, 25P, 26V, 31A;
(16) 12L, 25S, 26Q, 33E;
(17) 12A, 19C, 25C, 26M, 37N;
(18) 12T, 25A, 31H, 37N;
(19) 12T, 25A, 37N, 45L; or
(20) 12T, 25C, 29L, 35L, 37N;
wherein, optionally, said sets of substitutions (1)-(6), (8)-(12) and (17)-(20) are relative to SEQ ID NO:5 (hMeCP2); set of substitution (13) is relative to SEQ ID NO:3 (hMBD3); and sets of substitutions (7) and (14)-(16) are relative to SEQ ID NO:2 (hMBD2).

3. The isolated MBD variant of claim 1 or 2, wherein
(1) the MBD core domain comprises any one or more, preferably at least 2, more preferably at least 3, most preferably all 4, of the amino acids 14R/K, 24D/E, 36R/K and 40E/Q/D using the positional numbering of SEQ ID NO:1; and/or
(2) wherein the MBD core domain comprises any one or more, preferably at least 4, more preferably at least 6, even more preferably at least 8, most preferably at least 10, of the amino acids 1P/K, 3L/V, 6G/D, 7W/F, 8R/Q/K/E/T, 9R/K, 17G, 27Y/F/L, 30P, 32G, 44Y/F and 45L/I using the positional numbering of SEQ ID NO:1; and/or
(3) the MBD core domain comprises any one or more, preferably at least 5, more preferably at least 10, even more preferably at least 15, most preferably at least 20, of the amino acids 1P/K, 2A/S/T, 3L/V, 4G/P, 5P/Q/C/E, 6G/D, 7W/F, 8R/Q/K/E/T, 9R/K, 10R/E/V/K, 11E/V/L, 12V/K, 13F/I/P/Q, 14R/K, 15K/R/L, 16F/S, 17G, 18A/L/K/R, 19T/S, 20C/A, 21G, 22R/K/H, 23S/R/F/Y, 24D/E, 25T/V, 26Y/F, 27Y/F/L, 28Q/F/Y/I, 29S/N/L, 30P, 31T/S/Q/D/A/H, 32G, 33D/K/L/E, 34R/K/A, 35I/F, 36R/K, 37S, 38K, 39V/P/S, 40E/Q/D/S, 41L, 42T/A/I, 43R/N/A, 44Y/F/V, 45L/I/F.

4. The isolated MBD variant of any one of claims 1 to 3, wherein the isolated MBD variant further comprises any one or more of the following substitutions using the positional numbering of SEQ ID NO:1: 29L, 31D/A/H, and 33E.

5. The isolated MBD variant of any one of claims 1 to 4, wherein the isolated MBD variant comprises one or more additional amino acid sequences N- and/or C-terminal to the MBD core domain, each preferably 1-80 amino acids in length, more preferably the additional N-terminal amino acid sequence being 2 to 20 amino acids in length, and/or the additional C-terminal amino acid sequence being 10 to 60 amino acids in length; optionally wherein the additional amino acid sequences N- and/or C-terminal to the MBD core domain correspond to the corresponding wildtype MBD as set forth in any one of SEQ ID Nos. 1-28 and comprise 10 or less, preferably 6 or less, substitutions relative to the corresponding wildtype MBD as set forth in any one of SEQ ID Nos. 1-28.

6. The isolated MBD variant of any one of claims 1 to 5, wherein
(A) the variant has an altered binding affinity, preferably increased affinity, for CpG dinucleotides and their complement in which
(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);
(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC);
(l) both cytosine bases are 5-carboxylated (caC);
(m) one cytosine base is 5-methylated cytosine (mC) and the other is non-modified (C); and/or
(n) both cytosine bases are 5-methylated (mC/mC); and/or
(B) the variant has differential binding affinity for any two CpG dinucleotides and their complement selected from the following oxidized 5-methylated cytosine configurations:
(a) one cytosine base is 5-hydroxymethylated cytosine (hmC) and the other is non-modified (C);
(b) one cytosine base is 5-hydroxymethylated cytosine (hmC) and the other is methylated (mC);
(c) both cytosine bases are 5-hydroxymethylated (hmC);
(d) one cytosine base is 5-hydroxymethylated cytosine (hmC) and the other is formylated (fC);
(e) one cytosine base is 5-hydroxymethylated cytosine (hmC) and the other is carboxylated (caC);
(f) one cytosine base is 5-formylated cytosine (fC) and the other is non-modified (C);
(g) one cytosine base is 5-formylated cytosine (fC) and the other is methylated (mC);
(h) both cytosine bases are 5-formylated (fC);
(i) one cytosine base is 5-formylated cytosine (fC) and the other is carboxylated (caC);
(j) one cytosine base is 5-carboxylated cytosine (caC) and the other is non-modified (C);
(k) one cytosine base is 5-carboxylated cytosine (caC) and the other is methylated (mC); and/or
(l) both cytosine bases are 5-carboxylated (caC).

7. The isolated MBD variant of any one of claims 1 to 6, comprising or consisting of any one of the amino acid sequences set forth in SEQ ID Nos. 46 to 67, preferably set forth in SEQ ID Nos. 46, 48, 52, 53, 55, 56 or 57.

8. Conjugate comprising the isolated MBD variant of any one of claims 1 to 7, wherein the conjugate optionally further comprises an enzyme, preferably a nuclease or methyltransferase, or a detectable label, preferably a fluorophore.

9. Use of the isolated MBD variant of any one of claims 1 to 7 or the conjugate of claim 8 for the determination of the methylation state of cytosine residues and/or oxidation state of 5-methylated cytosine residues in a CpG dinucleotide of interest and its complement in a DNA molecule or for the enrichment of DNA molecules comprising a CpG dinucleotide of interest and its complement in which at least one cytosine nucleobase in the CpG dinucleotide of interest is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC).

10. Method for the determination of the methylation state of cytosine residues and/or oxidation state of the methyl group of 5-methylated cytosine residues in a CpG dinucleotide of interest and its complement in a DNA molecule, said method comprising (a) providing a molecular probe comprising a Methyl-CpG binding domain (MBD) variant according to any one of claims 1 to 7 that binds to the region of the DNA molecule comprising said CpG dinucleotide of interest and its complement and differentially binds to different methylated cytosine and/or oxidized 5-methyl-cytosine configurations in the CpG dinucleotide of interest and its complement, wherein said differential binding is detectable by differences in binding affinity;
(b) determining the methylation state of said cytosine residues and/or oxidation state of said 5-methylated cytosine residues in said CpG dinucleotide of interest by contacting the molecular probe with the DNA molecule and determining the binding affinity of the molecular probe to the region of the DNA molecule comprising said CpG dinucleotide of interest.

11. The method of claim 10, wherein the determination of the methylation state of cytosine residues and/or oxidation state of the methyl group of 5-methylated cytosine residues in a CpG dinucleotide of interest and its complement in a DNA molecule comprises determining the presence or the level of a nucleobase selected from the group consisting of cytosine (C), 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), in the CpG dinucleotide of interest and its complement.

12. Method for the enrichment of DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), said method comprising
(a) providing a molecular probe comprising a Methyl-CpG binding domain (MBD) variant according to any one of claims 1 to 7 that binds to the region of the DNA molecule comprising said CpG dinucleotide of interest and differentially binds to different methylated cytosine and/or oxidized 5-methyl-cytosine configurations in the CpG dinucleotide of interest and its complement, wherein said differential binding is facilitated by differences in binding affinity;
(b) contacting the molecular probe with a sample comprising DNA molecules comprising said CpG dinucleotide of interest and its complement under conditions that allow binding of the molecular probe to its target;
(c) enriching the DNA molecules comprising a CpG dinucleotide of interest in which at least one cytosine nucleobase in the CpG dinucleotide of interest and its complement is modified and selected from the group consisting of 5-methylcytosine (mC), 5-hydroxymethylcytosine (hmC), 5-formylcytosine (fC), and 5-carboxylcytosine (caC), preferably consisting of 5-hydroxymethylcytosine (hmC), 5-formylcytosine (fC), and 5-carboxylcytosine (caC), by separating the DNA molecules based on their affinity for the molecular probe.

13. The method of claim 12, wherein
(1) the molecular probe is immobilized on a substrate, wherein the molecular probe optionally comprises an affinity ligand that allows immobilization of the molecular probe on a substrate; and/or
(2) the enrichment step comprises separating the complexes of the DNA molecules with the immobilized molecular probe from the non-complexed DNA molecules, the enrichment optionally including chromatography, centrifugation or magnetic bead separation.

14. The method of any one of claims 10 to 13, wherein the difference in binding affinity of the molecular probe is an increased binding affinity for a methylation state of a cytosine residue and/or an oxidized state of 5-methylated cytosine, preferably an oxidized state of 5-methylated cytosine, in particular 5-hydroxymethylcytosine, relative to the non-methylated cytosine and/or non-oxidized 5-methylated cytosine.

## Patentansprüche

1. Isolierte Methyl-CpG-Bindungsdomänen (MBD)-Variante, umfassend eine MBD-Kerndomäne, die mindestens 85% Sequenzidentität bezogen auf eine der SEQ ID Nos. 1-28 aufweist und mindestens eine Aminosäuresubstitution, bezogen auf die entsprechende Wildtyp-MBD gemäß einer von SEQ ID Nos. 1-28, in mindestens einer der Positionen, die den Positionen 12, 25, 26, und 37 in SEQ ID NO: 1 entsprechen, umfasst, wobei die mindestens eine, bevorzugt mindestens zwei oder mindestens drei Aminosäuresubstitutionen in mindestens einer, bevorzugt in mindestens zwei oder mindestens drei, der Positionen, die den Positionen 12, 25, 26 und 37 entsprechen, ausgewählt ist aus 12V, 12T, 12A, 12R, 12D, 12L, 12P, 25T, 25A, 25C, 25L, 25Y, 25P, 25S, 26T, 26S, 26F, 26L, 26D, 26V, 26Q, 26M, 37N, 37K, 37Q, 37R, 37V und 37F, bevorzugt 12V, 12T, 12A, 12R, 25T, 25A, 25C, 25L, 26T, 26S, 26F, 26L, 26Q, 37N, 37K, 37Q, 37R und 37V, am meisten bevorzugt 12V, 12T, 25T, 25A, 25C, 26T, 26S, 26L, 26Q, 37N, 37R, und 37K unter Verwendung der Positionsnummerierung von SEQ ID NO: 1, wobei die MBD-Variante bezogen auf die entsprechende Wildtyp-MBD eine veränderte Affinität für ein DNA-Molekül aufweist, das ein CpG-Dinukleotid von Interesse und dessen Komplement umfasst, in dem mindestens eine Cytosin-Nukleobase in dem CpG-Dinukleotid von Interesse modifiziert ist und ausgewählt ist aus der Gruppe bestehend aus 5-Methylcytosin (mC), 5-Hydroxymethylcytosin (hmC), 5-Formylcytosin (fC) und 5-Carboxylcytosin (caC), bevorzugt bestehend aus 5-Hydroxymethylcytosin (hmC), 5-Formylcytosin (fC) und 5-Carboxylcytosin (caC).

2. Die isolierte MBD-Variante gemäß Anspruch 1, wobei die Variante zwei oder mehr, bevorzugt drei oder alle, der Substitutionen gemäß den folgenden Sets von Substitutionen umfasst:
(1) 12T, 25T, 26T, 37K;
(2) 12T, 25A, 37N;
(3) 25C, 26S, 37Q;
(4) 12A, 25C, 26F, 37R;
(5) 25L, 26T, 37R;
(6) 12V, 26L, 37R;
(7) 12R, 37V;
(8) 12T, 25T, 26Q, 37K;
(9) 12T, 25C, 37N;
(10) 12T, 25A, 26M, 37N;
(11) 12D, 25C, 37N;
(12) 12A, 25L, 26M, 37R;
(13) 12L, 25Y, 27F, 37F;
(14) 12L, 25A, 26D, 31D:
(15) 12P, 25P, 26V, 31A;
(16) 12L, 25S, 26Q, 33E;
(17) 12A, 19C, 25C, 26M, 37N;
(18) 12T, 25A, 31H, 37N;
(19) 12T, 25A, 37N, 45L; oder
(20) 12T, 25C, 29L, 35L, 37N;
wobei sich gegebenenfalls die Substitutionssets (1)-(6), (8)-(12) und (17)-(20) auf SEQ ID NO:5 (hMeCP2); das Substitutionsset (13) auf SEQ ID NO:3 (hMBD3); und die Substitutionssets (7) und (14)-(16) auf SEQ ID NO:2 (hMBD2) beziehen.

3. Die isolierte MBD-Variante gemäß Anspruch 1 oder 2, wobei
(1) die MBD-Kerndomäne eine oder mehrere, bevorzugt mindestens 2, stärker bevorzugt mindestens 3, und am meisten bevorzugt alle 4, der Aminosäuren 14R/K, 24D/E, 36R/K und 40E/Q/D unter Verwendung der Positionsnummerierung von SEQ ID NO:1 umfasst; und/oder
(2) wobei die MBD-Kerndomäne eine oder mehrere, bevorzugt mindestens 4, stärker bevorzugt mindestens 6, noch stärker bevorzugt mindestens 8, am meisten bevorzugt mindestens 10 der Aminosäuren 1P/K, 3L/V, 6G/D, 7W/F, 8R/Q/K/E/T, 9R/K, 17G, 27Y/F/L, 30P, 32G, 44Y/F and 45L/I unter Verwendung der Positionsnummerierung von SEQ ID NO:1 umfasst; und/oder
(3) die MBD-Kerndomäne eine oder mehrere, bevorzugt mindestens 5, stärker bevorzugt mindestens 10, noch stärker bevorzugt mindestens 15, am meisten bevorzugt mindestens 20 der Aminosäuren 1P/K, 2A/S/T, 3L/V, 4G/P, 5P/Q/C/E, 6G/D, 7W/F, 8R/Q/K/E/T, 9R/K, 10R/E/V/K, 11E/V/L, 12V/K, 13F/I/P/Q, 14R/K, 15K/R/L, 16F/S, 17G, 18A/L/K/R, 19T/S, 20C/A, 21G, 22R/K/H, 23S/R/F/Y, 24D/E, 25T/V, 26Y/F, 27Y/F/L, 28Q/F/Y/I, 29S/N/L, 30P, 31T/S/Q/D/A/H, 32G, 33D/K/L/E, 34R/K/A, 35I/F, 36R/K, 37S, 38K, 39V/P/S, 40E/Q/D/S, 41L, 42T/A/I, 43R/N/A, 44Y/F/V, 45L/I/F umfasst.

4. Die isolierte MBD-Variante gemäß einem der Ansprüche 1 bis 3, wobei die isolierte MBD-Variante ferner eine oder mehrere der folgenden Substitutionen unter Verwendung der Positionsnummerierung von SEQ ID NO:1 umfasst: 29L, 31D/A/H, und 33E.

5. Die isolierte MBD-Variante gemäß einem der Ansprüche 1 bis 4, wobei die isolierte MBD-Variante eine oder mehrere zusätzliche Aminosäuresequenzen N- und/oder C-terminal zu der MBD-Kerndomäne umfasst, wobei jede bevorzugt 1-80 Aminosäuren lang ist, stärker bevorzugt ist die zusätzliche N-terminale Aminosäuresequenz 2 bis 20 Aminosäuren lang und/oder die zusätzliche C-terminale Aminosäuresequenz ist 10 bis 60 Aminosäuren lang;
wobei sich gegebenenfalls die zusätzliche Aminosäuresequenz N- und/oder C-terminal zu der MBD-Kerndomäne auf die entsprechende Wildtyp-MBD gemäß einer der SEQ ID Nos. 1-28 bezieht, und 10 oder weniger, bevorzugt 6 oder weniger, Substitutionen bezogen auf die entsprechende Wildtyp-MBD gemäß einer der SEQ ID Nos. 1-28 umfasst.

6. Die isolierte MBD-Variante gemäß einem der Ansprüche 1 bis 5, wobei
(A) die Variante eine veränderte Bindungsaffinität aufweist, bevorzugt eine erhöhte Affinität, für CpG-Dinukleotide und deren Komplement, in denen
(a) eine Cytosinbase 5-hydroxymethyliertes Cytosin (hmC) und die andere nicht-modifiziert (C) ist;
(b) eine Cytosinbase 5-hydroxymethyliertes Cytosin (hmC) und die andere methyliert (mC) ist;
(c) beide Cytosinbasen 5-hydroxymethyliert (hmC) sind;
(d) eine Cytosinbase 5-hydroxymethyliertes Cytosin (hmC) und die andere formyliert (fC) ist;
(e) eine Cytosinbase 5-hydroxymethyliertes Cytosin (hmC) und die andere carboxyliert (caC) ist;
(f) eine Cytosinbase 5-formyliertes Cytosin (fC) und die andere nicht-modifiziert (C) ist;
(g) eine Cytosinbase 5-formyliertes Cytosin (fC) und die andere methyliert (mC) ist;
(h) beide Cytosinbasen 5-formyliert (fC) sind;
(i) eine Cytosinbase 5-formyliertes Cytosin (fC) und die andere carboxyliert (caC) ist;
(j) eine Cytosinbase 5-carboxyliertes Cytosin (caC) und die andere nicht-modifiziert (C) ist;
(k) eine Cytosinbase 5-carboxyliertes Cytosin (caC) und die andere methyliert (mC) ist;
(l) beide Cytosinbasen 5-carboxyliert (CaC) sind;
(m) eine Cytosinbase 5-methyliertes Cytosin (mC) und die andere nicht-modifiziert (C) ist; und/oder
(n) beide Cytosinbasen 5-methyliert (mC/mC) sind; und/oder
(B) die Variante unterschiedliche Bindungsaffinitäten für zwei CpG-Dinukleotide und deren Komplement aufweist, ausgewählt aus den folgenden oxidierten 5-methylierten Cytosin-Konfigurationen:
(a) eine Cytosinbase ist 5-hydroxymethyliertes Cytosin (hmC) und die andere ist nicht-modifiziert (C);
(b) eine Cytosinbase ist 5-hydroxymethyliertes Cytosin (hmC) und die andere ist methyliert (mC);
(c) beide Cytosinbasen sind 5-hydroxymethyliert (hmC);
(d) eine Cytosinbase ist 5-hydroxymethyliertes Cytosin (hmC) und die andere ist formyliert (fC);
(e) eine Cytosinbase ist 5-hydroxymethyliertes Cytosin (hmC) und die andere ist carboxyliert (caC);
(f) eine Cytosinbase ist 5-formyliertes Cytosin (fC) und die andere ist nicht-modifiziert (C);
(g) eine Cytosinbase ist 5-formyliertes Cytosin (fC) und die andere ist methyliert (mC);
(h) beide Cytosinbasen sind 5-formyliert (fC);
(i) eine Cytosinbase ist 5-formyliertes Cytosin (fC) und die andere ist carboxyliert (caC);
(j) eine Cytosinbase ist 5-carboxyliertes Cytosin (caC) und die andere ist nicht-modifiziert (C);
(k) eine Cytosinbase ist 5-carboxyliertes Cytosin (caC) und die andere ist methyliert (mC);
(l) beide Cytosinbasen sind 5-carboxyliert (CaC).

7. Die isolierte MBD-Variante gemäß einem der Ansprüche 1 bis 6, umfassend oder bestehend aus einer der Aminosäuresequenzen gemäß SEQ **ID** Nos. 46 bis 67, bevorzugt gemäß SEQ **ID** Nos. 46, 48, 52, 53, 55, 56 oder 57.

8. Konjugat, umfassend die isolierte MBD-Variante gemäß einem der Ansprüche 1 bis 7, wobei das Konjugat gegebenenfalls ferner ein Enzym, bevorzugt eine Nuklease oder Methyltransferase, oder eine detektierbare Markierung, bevorzugt ein Fluorophor, umfasst.

9. Verwendung der isolierten MBD-Variante gemäß einem der Ansprüche 1 bis 7 oder des Konjugats gemäß Anspruch 8 zur Bestimmung des Methylierungszustands der Cytosinreste und/oder des Oxidationszustands der 5-methylierten Cytosinreste in einem CpG-Dinukleotid von Interesse und seines Komplements in einem DNA-Molekül oder für die Anreicherung von DNA-Molekülen umfassend ein CpG-Dinukleotid von Interesse oder sein Komplement in welchem mindestens eine Cytosin-Nukleobase in dem CpG-Dinukleotid von Interesse modifiziert ist und ausgewählt wird aus der Gruppe bestehend aus 5-Methylcytosin (mC), 5-Hydroxymethylcytosin (hmC), 5-Formylcytosin (fC) und 5-Carboxylcytosin (caC), bevorzugt bestehend aus 5-Hydroxymethylcytosin (hmC), 5-Formylcytosin (fC) und 5-Carboxylcytosin (caC).

10. Verfahren zur Bestimmung des Methylierungszustands der Cytosinreste und/oder des Oxidationszustands der Methylgruppe der 5-methylierten Cytosinreste in einem CpG-Dinukleotid von Interesse und dessen Komplement in einem DNA-Molekül, wobei das Verfahren umfasst
(a) das Bereitstellen einer molekularen Sonde umfassend eine Methyl-CpG-Bindungsdomänen (MBD)-Variante gemäß einem der Ansprüche 1 bis 7, die an die Region des DNA-Moleküls bindet, das das CpG-Dinukleotid von Interesse und dessen Komplement umfasst und unterschiedlich an die unterschiedlich methylierten Cytosine und/oder oxidierten 5-Methyl-Cytosin-Konfigurationen in dem CpG-Dinukleotid von Interesse und dessen Komplement bindet, wobei die unterschiedliche Bindung durch Unterschiede in der Bindungsaffinität detektierbar ist;
(b) das Bestimmen des Methylierungszustands der Cytosinreste und/oder des Oxidationszustandes der 5-methylierten Cytosinreste in dem CpG-Dinukleotid von Interesse, durch Kontaktieren der molekularen Sonde mit dem DNA-Molekül und Bestimmen der Bindungsaffinität der molekularen Sonde an die Region des DNA-Moleküls umfassend das CpG-Dinukleotid von Interesse.

11. Das Verfahren gemäß Anspruch 10, wobei das Bestimmen des Methylierungszustands der Cytosinreste und/oder des Oxidationszustands der Methylgruppe der 5-methylierten Cytosinreste in einem CpG-Dinukleotid von Interesse und dessen Komplement in einem DNA-Molekül das Bestimmen des Vorliegens oder des Levels einer Nukleobase ausgewählt aus der Gruppe bestehend aus Cytosin (C), 5-Methylcytosin (mC), 5-Hydroxymethylcytosin (hmC), 5-Formylcytosin (fC) und 5-Carboxylcytosin (caC), bevorzugt bestehend aus 5-Hydroxymethylcytosin (hmC), 5-Formylcytosin (fC) und 5-Carboxylcytosin (caC), in dem CpG-Dinukleotid von Interesse und dessen Komplement umfasst.

12. Das Verfahren zur Anreicherung von DNA-Molekülen umfassend ein CpG-Dinukleotid von Interesse, in dem mindestens eine Cytosinnukleobase in dem CpG-Dinukleotid von Interesse und dessen Komplement modifiziert ist und ausgewählt ist aus der Gruppe bestehend aus 5-Methylcytosin (mC), 5-Hydroxymethylcytosin (hmC), 5-Formylcytosin (fC) und 5-Carboxylcytosin (caC), bevorzugt bestehend aus 5-Hydroxymethylcytosin (hmC), 5-Formylcytosin (fC) und 5-Carboxylcytosin (caC), wobei das Verfahren umfasst
(a) das Bereitstellen einer molekularen Sonde umfassend eine Methyl-CpG-Bindungsdomänen (MBD)-Variante gemäß einem der Ansprüche 1 bis 7, die an die Region des DNA-Moleküls bindet, das das CpG-Dinukleotid von Interesse umfasst und unterschiedlich an die unterschiedlich methylierten Cytosine und/oder oxidierten 5-Methyl-Cytosin-Konfigurationen in dem CpG-Dinukleotid von Interesse und dessen Komplement bindet, wobei die unterschiedliche Bindung durch Unterschiede in der Bindungsaffinität erleichtert wird;
(b) das Kontaktieren der molekularen Sonde mit einer Probe umfassend DNA-Moleküle umfassend das CpG-Dinukleotid von Interesse und dessen Komplement unter Bedingungen die die Bindung der molekularen Sonde an ihr Ziel erlauben;
(c) das Anreichern der DNA-Moleküle umfassend ein CpG-Dinukleotid von Interesse in dem mindestens eine Cytosin-Nukleobase in dem CpG-Dinukleotid von Interesse oder dessen Komplement modifiziert ist und ausgewählt ist aus der Gruppe bestehend aus 5-Methylcytosin (mC), 5-Hydroxymethylcytosin (hmC), 5-Formylcytosin (fC) und 5-Carboxylcytosin (caC), bevorzugt bestehend aus 5-Hydroxymethylcytosin (hmC), 5-Formylcytosin (fC) und 5-Carboxylcytosin (caC), durch Trennen der DNA-Moleküle basierend auf ihrer Affinität für die molekulare Sonde.

13. Das Verfahren gemäß Anspruch 12, wobei
(1) die molekulare Sonde auf einem Substrat immobilisiert ist, wobei die molekulare Sonde gegebenenfalls einen Affinitätsliganden umfasst, der die Immobilisierung der molekularen Sonde auf einem Substrat erlaubt; und/oder
(2) der Anreicherungsschritt das Trennen der Komplexe der DNA-Moleküle mit der immobilisierten molekularen Sonde von den nicht-komplexierten DNA-Molekülen umfasst, wobei das Anreichern gegebenenfalls Chromatographie, Zentrifugation oder Trennung mit magnetischen Beads umfasst.

14. Das Verfahren gemäß einem der Ansprüche 10 bis 13, wobei der Unterschied in der Bindungsaffinität der molekularen Sonde eine erhöhte Bindungsaffinität für einen Methylierungszustand eines Cytosinrestes und/oder eines Oxidationszustands eines 5-methylierten Cytosins ist, bevorzugt ein Oxidationszustand von 5-methyliertem Cytosin, insbesondere 5-Hydroxymethylcytosin, bezogen auf das nicht-methylierte Cytosin und/oder das nicht-oxidierte 5-methylierte Cytosin.

## Revendications

1. Variant isolé de domaine de liaison au CpG méthylé (MBD), comprenant un domaine central de MBD qui présente au moins 85 % d'identité de séquence par rapport à l'une quelconque de SEQ ID N^{os} 1 à 28 et comprenant au moins une substitution d'acide aminé par rapport au MBD de type sauvage correspondant tel que défini dans l'une quelconque de SEQ ID N^{os} 1 à 28 à au moins une des positions correspondant aux positions 12, 25, 26 et 37, dans SEQ ID N° 1, sachant que ladite au moins une, de préférence lesdites au moins deux ou au moins trois, substitution(s) d'acide aminé dans au moins une, de préférence dans au moins deux ou au moins trois, des positions correspondant aux positions 12, 25, 26 et 37 est sélectionnée parmi 12V, 12T, 12A, 12R, 12D, 12L, 12P, 25T, 25A, 25C, 25L, 25Y, 25P, 25S, 26T, 26S, 26F, 26L, 26D, 26V, 26Q, 26M, 37M, 37K, 37Q, 37R, 37V et 37F, de préférence 12V, 12T, 12A, 12R, 25T, 25A, 25C, 25L, 26T, 26S, 26F, 26L, 26Q, 37N, 37K, 37Q, 37R et 37V, et de manière la plus préférable 12V, 12T, 25T, 25A, 25C, 26T, 26S, 26L, 26Q, 37N, 37R, et 37K selon la numérotation des positions de SEQ ID N° 1, sachant que ledit variant de MBD présente, par rapport au MBD de type sauvage correspondant, une affinité modifiée pour une molécule d'ADN comprenant un dinucléotide CpG d'intérêt et son complément dans lequel au moins une nucléobase cytosine dans le dinucléotide CpG d'intérêt est modifiée et sélectionnée parmi le groupe constitué d'une 5-méthylcytosine (mC), d'une 5-hydroxyméthylcytosine (hmC), d'une 5-formylcytosine (fC), et d'une 5-carboxylcytosine (caC), de préférence constitué d'une 5-hydroxyméthylcytosine (hmC), d'une 5-formylcytosine (fC), et d'une 5-carboxylcytosine (caC).

2. Le variant isolé de MBD de la revendication 1, sachant que ledit variant comprend deux quelconques ou plusieurs, de préférence trois ou l'ensemble, des substitutions définies dans les ensembles de substitutions suivants :
(1) 12T, 25T, 26T, 37K ;
(2) 12T, 25A, 37N ;
(3) 25C, 26S, 37Q ;
(4) 12A, 25C, 26F, 37R ;
(5) 25L, 26T, 37R ;
(6) 12V, 26L, 37R ;
(7) 12R, 37V ;
(8) 12T, 25T, 26Q, 37K ;
(9) 12T, 25C, 37N ;
(10) 12T, 25A, 26M, 37N ;
(11) 12D, 25C, 37N ;
(12) 12A, 25L, 26M, 37R ;
(13) 12L, 25Y, 27F, 37F ;
(14) 12L, 25A, 26D, 31D ;
(15) 12P, 25P, 26V, 31A ;
(16) 12L, 25S, 26Q, 33E ;
(17) 12A, 19C, 25C, 26M, 37N ;
(18) 12T, 25A, 31H, 37N ;
(19) 12T, 25A, 37N, 45L ; ou
(20) 12T, 25C, 29L, 35L, 37N ;
sachant que, facultativement, lesdits ensembles de substitutions (1) à (6), (8) à (12) et (17) à (20) se rapportent à SEQ ID N° 5 (hMeCP2) ; l'ensemble de substitution (13) se rapporte à SEQ ID N° 3 (hMBD3) ; et les ensembles de substitutions (7) et (14) à (16) se rapportent à SEQ ID N° 2 (hMBD2).

3. Le variant isolé de MBD de la revendication 1 ou 2, sachant que
(1) le domaine central de MBD comprend un quelconque ou plusieurs, de préférence au moins 2, plus préférablement au moins 3, et de manière la plus préférable l'ensemble des 4, des acides aminés 14R/K, 24D/E, 36R/K et 40E/Q/D selon la numérotation des positions de SEQ ID N° 1 ; et/ou
(2) sachant que le domaine central de MBD comprend un quelconque ou plusieurs, de préférence au moins 4, plus préférablement au moins 6, encore plus préférablement au moins 8, et de manière la plus préférable au moins 10, des acides aminés 1P/K, 3L/V, 6G/D, 7W/F, 8R/Q/K/E/T, 9R/K, 17G, 27Y/F/L, 30P, 32G, 44Y/F et 45L/I selon la numérotation des positions de SEQ ID N° 1 ; et/ou
(3) le domaine central de MBD comprend un quelconque ou plusieurs, de préférence au moins 5, plus préférablement au moins 10, encore plus préférablement au moins 15, et de manière la plus préférable au moins 20, des acides aminés 1P/K, 2A/S/T, 3L/V, 4G/P, 5P/Q/C/E, 6G/D, 7W/F, 8R/Q/K/E/T, 9R/K, 10R/E/V/K, 11E/V/L, 12V/K, 13F/I/P/Q, 14R/K, 15K/R/L, 16F/S, 17G, 18A/L/K/R, 19T/S, 20C/A, 21G, 22R/K/H, 23S/R/F/Y, 24D/E, 25T/V, 26Y/F, 27Y/F/L, 28Q/F/Y/I, 29S/N/L, 30P, 31T/S/Q/D/A/H, 32G, 33D/K/L/E, 34R/K/A, 35I/F, 36R/K, 37S, 38K, 39V/P/S, 40E/Q/D/S, 41L, 42T/A/I, 43R/N/A, 44Y/F/V, 45L/I/F.

4. Le variant isolé de MBD selon l'une quelconque des revendications 1 à 3, sachant que le variant isolé de MBD comprend en outre une quelconque ou plusieurs des substitutions suivantes selon la numérotation des positions de SEQ ID N° 1 : 29L, 31D/A/H, et 33E.

5. Le variant isolé de MBD de l'une quelconque des revendications 1 à 4, sachant que le variant isolé de MBD comprend une ou plusieurs séquences d'acides aminés supplémentaires en position N-terminale et/ou C-terminale par rapport au domaine central de MBD, chacune ayant de préférence une longueur de 1 à 80 acides aminés, plus préférablement la séquence d'acides aminés supplémentaire N-terminale ayant une longueur de 2 à 20 acides aminés, et/ou la séquence d'acides aminés supplémentaire C-terminale ayant une longueur de 10 à 60 acides aminés ; facultativement sachant que les séquences d'acides aminés supplémentaires en position N-terminale et/ou C-terminale par rapport au domaine central de MBD correspondent au MBD de type sauvage correspondant tel que défini dans l'une quelconque de SEQ ID N^{os} 1 à 28 et comprennent 10 ou moins, de préférence 6 ou moins, substitutions par rapport au MBD de type sauvage correspondant tel que défini dans l'une quelconque de SEQ ID N^{os} 1 à 28.

6. Le variant isolé de MBD de l'une quelconque des revendications 1 à 5, sachant que
(A) le variant présente une affinité de liaison modifiée, de préférence une affinité accrue, pour des dinucléotides CpG et leur complément dans lesquels
(a) une base cytosine est une cytosine 5-hydroxyméthylée (hmC) et l'autre est non modifiée (C) ;
(b) une base cytosine est une cytosine 5-hydroxyméthylée (hmC) et l'autre est méthylée (mC) ;
(c) les deux bases cytosine sont 5-hydroxyméthylées (hmC) ;
(d) une base cytosine est une cytosine 5-hydroxyméthylée (hmC) et l'autre est formylée (fC) ;
(e) une base cytosine est une cytosine 5-hydroxyméthylée (hmC) et l'autre est carboxylée (caC) ;
(f) une base cytosine est une cytosine 5-formylée (fC) et l'autre est non modifiée (C) ;
(g) une base cytosine est une cytosine 5-formylée (fC) et l'autre est méthylée (mC) ;
(h) les deux bases cytosine sont 5-formylées (fC) ;
(i) une base cytosine est une cytosine 5-formylée (fC) et l'autre est carboxylée (caC) ;
(j) une base cytosine est une cytosine 5-carboxylée (caC) et l'autre est non modifiée (C) ;
(k) une base cytosine est une cytosine 5-carboxylée (caC) et l'autre est méthylée (mC) ;
(l) les deux bases cytosine sont 5-carboxylées (caC) ;
(m) une base cytosine est une cytosine 5-méthylée (mC) et l'autre est non modifiée (C) ; et/ou
(n) les deux bases cytosine sont 5-méthylées (mC/mC) ; et/ou
(B) le variant présente une affinité de liaison différentielle pour deux quelconques dinucléotides CpG et leur complément sélectionnés parmi les configurations suivantes de cytosine oxydée 5-méthylée :
(a) une base cytosine est une cytosine 5-hydroxyméthylée (hmC) et l'autre est non modifiée (C) ;
(b) une base cytosine est une cytosine 5-hydroxyméthylée (hmC) et l'autre est méthylée (mC) ;
(c) les deux bases cytosine sont 5-hydroxyméthylées (hmC) ;
(d) une base cytosine est une cytosine 5-hydroxyméthylée (hmC) et l'autre est formylée (fC) ;
e) une base cytosine est une cytosine 5-hydroxyméthylée (hmC) et l'autre est carboxylée (caC) ;
f) une base cytosine est une cytosine 5-formylée (fC) et l'autre est non modifiée (C) ;
g) une base cytosine est une cytosine 5-formylée (fC) et l'autre est méthylée (mC) ;
h) les deux bases cytosine sont 5-formylées (fC) ;
i) une base cytosine est une cytosine 5-formylée (fC) et l'autre est carboxylée (caC) ;
j) une base cytosine est une cytosine 5-carboxylée (caC) et l'autre est non modifiée (C) ;
k) une base cytosine est une cytosine 5-carboxylée (caC) et l'autre est méthylée (mC) ; et/ou
l) les deux bases cytosine sont 5-carboxylées (caC).

7. Le variant isolé de MBD de l'une quelconque des revendications 1 à 6, comprenant ou constitué de l'une quelconque des séquences d'acides aminés définies dans SEQ ID N^{os} 46 à 67, de préférence définies dans SEQ ID N^{os} 46, 48, 52, 53, 55, 56 ou 57.

8. Conjugué comprenant le variant isolé de MBD de l'une quelconque des revendications 1 à 7, sachant que le conjugué comprend en outre facultativement une enzyme, de préférence une nucléase ou une méthyltransférase, ou un marqueur détectable, de préférence un fluorophore.

9. Utilisation du variant isolé de MBD de l'une quelconque des revendications 1 à 7 ou du conjugué de la revendication 8 pour la détermination de l'état de méthylation de résidus de cytosine et/ou de l'état d'oxydation de résidus de cytosine 5-méthylée dans un dinucléotide CpG d'intérêt et son complément dans une molécule d'ADN ou pour l'enrichissement de molécules d'ADN comprenant un dinucléotide CpG d'intérêt et son complément dans lequel au moins une nucléobase cytosine dans le dinucléotide CpG d'intérêt est modifiée et sélectionnée parmi le groupe constitué d'une 5-méthylcytosine (mC), d'une 5-hydroxyméthylcytosine (hmC), d'une 5-formylcytosine (fC), et d'une 5-carboxylcytosine (caC), de préférence constitué d'une 5-hydroxyméthylcytosine (hmC), d'une 5-formylcytosine (fC), et d'une 5-carboxylcytosine (caC).

10. Procédé de détermination de l'état de méthylation de résidus de cytosine et/ou de l'état d'oxydation du groupe méthyle de résidus de cytosine 5-méthylée dans un dinucléotide CpG d'intérêt et son complément dans une molécule d'ADN, ledit procédé comprenant
(a) la fourniture d'une sonde moléculaire comprenant un variant de domaine de liaison au CpG méthylé (MBD) selon l'une quelconque des revendications 1 à 7 qui se lie à la région de la molécule d'ADN comprenant ledit dinucléotide CpG d'intérêt et son complément et se lie de manière différentielle à différentes configurations de cytosine méthylée et/ou de 5-méthyl-cytosine oxydée dans le dinucléotide CpG d'intérêt et son complément, sachant que ladite liaison différentielle est détectable par des différences d'affinité de liaison ;
(b) la détermination de l'état de méthylation desdits résidus de cytosine et/ou de l'état d'oxydation desdits résidus de cytosine 5-méthylée dans ledit dinucléotide CpG d'intérêt et son complément par mise en contact de la sonde moléculaire avec la molécule d'ADN et détermination de l'affinité de liaison de la sonde moléculaire à la région de la molécule d'ADN comprenant ledit dinucléotide CpG d'intérêt.

11. Le procédé de la revendication 10, sachant que la détermination de l'état de méthylation de résidus de cytosine et/ou de l'état d'oxydation du groupe méthyle de résidus de cytosine 5-méthylée dans un dinucléotide CpG d'intérêt et son complément dans une molécule d'ADN comprend la détermination de la présence ou du niveau d'une nucléobase sélectionnée parmi le groupe constitué d'une cytosine (C), d'une 5-méthylcytosine (mC), d'une 5-hydroxyméthylcytosine (hmC), d'une 5-formylcytosine (fC), et d'une 5-carboxylcytosine (caC), de préférence constitué d'une 5-hydroxyméthylcytosine (hmC), d'une 5-formylcytosine (fC), et d'une 5-carboxylcytosine (caC), dans le dinucléotide CpG d'intérêt et son complément.

12. Procédé d'enrichissement de molécules d'ADN comprenant un dinucléotide CpG d'intérêt dans lequel au moins une nucléobase cytosine dans le dinucléotide CpG d'intérêt et son complément est modifiée et sélectionnée parmi le groupe constitué d'une 5-méthylcytosine (mC), d'une 5-hydroxyméthylcytosine (hmC), d'une 5-formylcytosine (fC), et d'une 5-carboxylcytosine (caC), de préférence constitué d'une 5-hydroxyméthylcytosine (hmC), d'une 5-formylcytosine (fC), et d'une 5-carboxylcytosine (caC), ledit procédé comprenant
(a) la fourniture d'une sonde moléculaire comprenant un variant de domaine de liaison au CpG méthylé (MBD) selon l'une quelconque des revendications 1 à 7 qui se lie à la région de la molécule d'ADN comprenant ledit dinucléotide CpG d'intérêt et se lie de manière différentielle à différentes configurations de cytosine méthylée et/ou de 5-méthyl-cytosine oxydée dans le dinucléotide CpG d'intérêt et son complément, sachant que ladite liaison différentielle est facilitée par des différences d'affinité de liaison ;
(b) la mise en contact de la sonde moléculaire avec un échantillon comprenant des molécules d'ADN comprenant ledit dinucléotide CpG d'intérêt et son complément dans des conditions permettant la liaison de la sonde moléculaire à sa cible ;
(c) l'enrichissement des molécules d'ADN comprenant un dinucléotide CpG d'intérêt dans lequel au moins une nucléobase cytosine dans le dinucléotide CpG d'intérêt et son complément est modifiée et sélectionnée parmi le groupe constitué d'une 5-méthylcytosine (mC), d'une 5-hydroxyméthylcytosine (hmC), d'une 5-formylcytosine (fC), et d'une 5-carboxylcytosine (caC), de préférence constitué d'une 5-hydroxyméthylcytosine (hmC), d'une 5-formylcytosine (fC), et d'une 5-carboxylcytosine (caC), par séparation des molécules d'ADN selon leur affinité pour la sonde moléculaire.

13. Le procédé de la revendication 12, sachant que
(1) la sonde moléculaire est immobilisée sur un substrat, sachant que la sonde moléculaire comprend facultativement un ligand d'affinité permettant l'immobilisation de la sonde moléculaire sur un substrat ; et/ou
(2) l'étape d'enrichissement comprend la séparation des complexes des molécules d'ADN avec la sonde moléculaire immobilisée par rapport aux molécules d'ADN non complexées, l'enrichissement incluant facultativement une chromatographie, une centrifugation ou une séparation sur billes magnétiques.

14. Le procédé de l'une quelconque des revendications 10 à 13, sachant que la différence d'affinité de liaison de la sonde moléculaire est une affinité de liaison accrue pour un état de méthylation d'un résidu de cytosine et/ou un état oxydé d'une cytosine 5-méthylée, de préférence un état oxydé d'une cytosine 5-méthylée, en particulier d'une 5-hydroxyméthylcytosine, par rapport à la cytosine non méthylée et/ou à la cytosine 5-méthylée non oxydée.
